# EUROPÄISCHE PATENTANMELDUNG

(11) **EP 0 855 392 A2**
(43) Veröffentlichungstag der Anmeldung: **29.07.1998**
(21) Anmeldenummer: 98100595.2
(22) Anmeldetag: 15.01.1998
(51) Int. Cl.: C07D 233/68, C07D 233/70, C07D 233/61, C07D 233/84, C07D 233/90, A61K 31/415

(54) **Fünfgliedrige Heterocyclen mit Biphenylsulfonylsubstitution, Verfahren zu ihrer Herstellung, ihre Verwendung als Medikament oder Diagnostikum sowie sie enthaltendes Medikament**

(30) Priorität: 22.01.1997 DE 19702045; 22.07.1997 DE 19731328; 22.09.1997 DE 19741636
(71) Anmelder: HOECHST AKTIENGESELLSCHAFT, 65929 Frankfurt am Main (DE)
(72) Erfinder: Kleemann, Heinz-Werner, Dr., 65474 Bischofsheim (DE); Lang, Hans Jochen, Dr., 65719 Hofheim (DE); Schwark, Jan-Robert, Dr., 65929 Frankfurt (DE); Weichert, Andreas, Dr., 63329 Egelsbach (DE); Faber, Sabine, AP, 65510 Idstein (DE); Jansen, Hans-Willi, Dr., 65527 Niedernhausen (DE)

(57) **Zusammenfassung**

Verbindungen der Formel I, 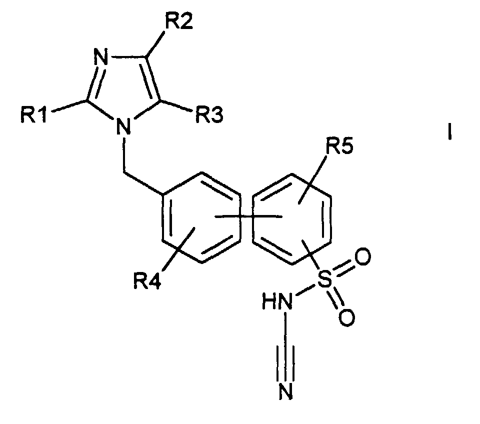 in welcher die Symbole die in den Ansprüchen angegebenen Bedeutungen haben, haben hervorragende antiarrhythmische Eigenschaften, sie weisen eine cardioprotektive Komponente auf. Sie können präventiv die pathophysiologischen Vorgänge beim Entstehen ischämisch induzierter Schäden, insbesondere bei der Auslösung ischämisch induzierter Herzarrhythmien, inhibieren oder stark vermindern. Darüber hinaus haben sie ein starke inhibierende Wirkung auf die Proliferationen von Zellen.

## Beschreibung

Die Erfindung betrifft Verbindungen der Formel I, 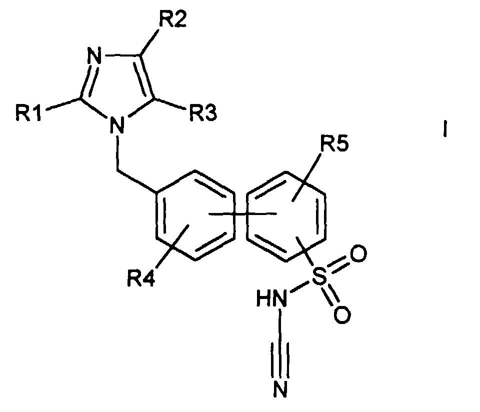 in welcher die Symbole folgende Bedeutung haben:
- R1: Wasserstoff, Alkyl mit 1, 2, 3, 4, 5, 6, 7 oder 8 C-Atomen oder -CₐH₂ₐ-Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(8)R(9);
- R(8) und R(9): unabhängig voneinander H oder (C₁-C₄)-Alkyl;
- a: Null, 1 oder 2;
oder
- R1: -C_{b}H_{2b}C₁-C₉)-Heteroaryl, das unsubstituiert oder substituiert ist mit 1 - 3 Substituenten ausgewählt aus der Gruppe bestehend aus F, Cl, Br, I, CF₃, CH₃, Methoxy, Hydroxy und NR(10)R(11);
- R(10) und R(11): unabhängig voneinander H oder (C₁-C₄)-Alkyl;
- b: Null, 1 oder 2;
oder
- R1: -C_{d}H_{2d}-(C₃-C₇)Cycloalkyl;
- d: Null, 1 oder 2;
- R2 und R3: unabhängig voneinander Wasserstoff, F, Cl, Br, I, CF₃, -C≡N, -NO₂,
CH₂OR17, CO-R6 oder O-R7;
- R17: Wasserstoff oder Alkyl mit 1, 2, 3, 4, 5, 6, 7 oder 8 C-Atomen
- R6: Wasserstoff, Alkyl mit 1, 2, 3, 4, 5, 6, 7 oder 8 C-Atomen, OR30 oder Phenyl,
das unsubstituiert oder substituiert ist mit 1 - 3 Substituenten ausgewählt aus der Gruppe bestehend aus bestehend aus F, Cl, Br, I, CF₃, Methyl, Methoxy, Hydroxy und NR(31)R(32); R(31) und R(32)
unabhängig voneinander H oder (C₁-C₄)-Alkyl;
- R30: Wasserstoff oder Alkyl mit 1, 2, 3, 4, 5, 6, 7 oder 8 C-Atomen;
- R7: Wasserstoff, Alkyl mit 1, 2, 3, 4, 5, 6, 7 oder 8 C-Atomen, Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(12)R(13);
- R(12) und R(13): unabhängig voneinander H oder (C₁-C₄)-Alkyl;
oder
- R7: (C₁-C₉)-Heteroaryl,
das unsubstituiert oder substituiert ist mit 1 - 3 Substituenten ausgewählt aus der Gruppe bestehend aus F, Cl, Br, I, CF₃, CH₃, Methoxy, Hydroxy und NR(14)R(15);
- R(14) und R(15): unabhängig voneinander H oder (C₁-C₄)-Alkyl;
oder
- R2 und R3: unabhängig voneinander Alkyl mit 1, 2, 3, 4, 5, 6, 7 oder 8 C-Atomen, Cycloalkyl mit 3, 4, 5, 6 oder 7 C-Atomen oder -C_{g}H₂g-Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(18)R(19);
- R(18) und R(19): unabhängig voneinander H oder (C₁-C₄)-Alkyl;
- g: Null, 1 oder 2;
oder
- R2 und R3: unabhängig voneinander -CₗH₂ₗ-(C₁-C₉)-Heteroaryl,
das unsubstituiert oder substituiert ist mit 1 - 3 Substituenten ausgewählt aus der Gruppe bestehend aus F, Cl, Br, I, CF₃, CH₃, Methoxy, Hydroxy und NR(20)R(21);
- R(20) und R(21): unabhängig voneinander H oder (C₁-C₄)-Alkyl;
- l: Null, 1 oder 2;
oder
- R2 und R3: unabhängig voneinander SOₙ-R22;
- n: Null, 1 oder 2;
- R22: Alkyl mit 1, 2, 3, 4, 5, 6, 7 oder 8 C-Atomen, Cycloalkyl mit 3, 4, 5, 6 oder 7 C-Atomen oder -CₛH₂ₛ-Phenyl,
das unsubstituiert oder substituiert ist mit 1 - 3 Substituenten ausgewählt aus der Gruppe bestehend Gruppe F, Cl, Br, I, CF₃, Methyl, Methoxy, Hydroxy und NR(34)R(35);
- R(34) und R(35): unabhängig voneinander H oder (C₁-C₄)-Alkyl;
- s: Null, 1 oder 2;
- R4 und R5: unabhängig voneinander Wasserstoff, Alkyl mit 1, 2, 3, 4, 5, 6, 7 oder 8 C-Atomen, F, Cl, Br, I, CF₃, -C≡N, -NO₂, SOₚ-R16, CO-R23 oder O-R24;
- p: Null, 1 oder 2;
- R16: Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, Br, I, CF₃, Methyl, Methoxy, Hydroxy und NR(26)R(27);
- R(26) und R(27): unabhängig voneinander H oder (C₁-C₄)-Alkyl;
- R23: Wasserstoff, Alkyl mit 1, 2, 3, 4, 5, 6, 7 oder 8 C-Atomen oder OR25; R25 Wasserstoff, Alkyl mit 1, 2, 3, 4, 5, 6, 7 oder 8 C-Atomen;
- R24: Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, Br, I, CF₃, Methyl, Methoxy, Hydroxy und NR(28)R(29);
- R(28) und R(29): unabhängig voneinander H oder (C₁-C₄)-Alkyl;
sowie deren physiologisch verträgliche Salze.

Bevorzugt sind Verbindungen der Formel I, in der bedeuten:
- R1: Wasserstoff, Alkyl mit 1, 2, 3 oder 4 C-Atomen oder -CₐH₂ₐ-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(8)R(9);
- R(8) und R(9): unabhängig voneinander H oder Methyl;
- a: Null oder 1;
oder
- R1: (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(10)R(11);
- R(10) und R(11): unabhängig voneinander H oder Methyl;
oder
- R1: -C_{d}H_{2d}-(C₃-C₇)-Cycloalkyl;
- d: Null oder 1;
- R2 und R3: unabhängig voneinander Wasserstoff, F, Cl, Br, CF₃, -C≡N, -NO₂, CH₂OR17, CO-R6 oder O-R7;
- R17: Wasserstoff oder Alkyl mit 1, 2, 3 oder 4 C-Atomen;
- R6: Wasserstoff, Alkyl mit 1, 2, 3 oder 4 C-Atomen, OR30 oder Phenyl, das unsubstituiert oder substituiert ist mit 1 - 2 Substituenten ausgewählt aus der Gruppe bestehend aus F, Cl, Br, CF₃, Methyl, Methoxy, Hydroxy und NR(31)R(32);
- R(31) und R(32): unabhängig voneinander H oder Methyl;
- R30: Wasserstoff oder Alkyl mit 1, 2, 3 oder 4 C-Atomen;
- R7: Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, Br, CF₃, Methyl, Methoxy, Hydroxy und NR(12)R(13);
- R(12) und R(13): unabhängig voneinander H oder Methyl;
oder
- R7: (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(14)R(15);
- R(14) und R(15): unabhängig voneinander H oder Methyl;
oder
- R2 und R3: unabhängig voneinander Alkyl mit 1, 2, 3 oder 4 C-Atomen, Cycloalkyl mit 3, 4, 5, 6 oder 7 C-Atomen oder -C_{g}H_{2g}-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(18)R(19);
- R(18) und R(19): unabhängig voneinander H oder Methyl;
- g: Null oder 1;
oder
- R2 und R3: unabhängig voneinander (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(20)R(21);
- R(20) und R(21): unabhängig voneinander H oder Methyl;
oder
- R2 und R3: unabhängig voneinander SOₙ-R22,
- n: Null, 1 oder 2;
- R22: Alkyl mit 1, 2, 3 oder 4 C-Atomen, Cycloalkyl mit 3, 4, 5, 6 oder 7 C-Atomen oder -CₛH₂ₛ-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(34)R(35);
- R(34) und R(35): ausgewählt aus der Gruppe bestehend aus H oder Methyl;
- s: Null oder 1;
- R4 und R5: unabhängig voneinander Wasserstoff, Alkyl mit 1, 2, 3 oder 4 C-Atomen, F, Cl, Br, CF₃, -C≡N, -NO₂, SOₚ-R16, CO-R23 oder O-R24;
- p: Null, 1 oder 2;
- R¹⁶: Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(26)R(27);
- R(26) und R(27): unabhängig voneinander H oder Methyl;
- R23: Wasserstoff, Alkyl mit 1, 2, 3 oder 4 C-Atomen oder OR25;
- R25: Wasserstoff oder Alkyl mit 1, 2, 3 oder 4 C-Atomen;
- R24: Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, Br, CF₃, Methyl, Methoxy, Hydroxy und NR(28)R(29);
- R(28) und R(29): unabhängig voneinander H oder Methyl;
sowie deren physiologisch verträgliche Salze.

Besonders bevorzugt sind Verbindungen der Formel I, in welcher bedeuten:
- R1: Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(8)R(9);
- R(8) und R(9): unabhängig voneinander H oder Methyl;
oder
- R1: (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(10)R(11);
- R(10) und R(11): unabhängig voneinander H oder Methyl;
oder
- R1: (C₃-C₇)-Cycloalkyl;
- R2 und R3: unabhängig voneinander Wasserstoff, F, Cl, Br, CF₃, -C≡N, -NO₂, CO-R6 oder O-R7;
- R6: Wasserstoff, Alkyl mit 1, 2, 3 oder 4 C-Atomen, OR30 oder Phenyl, das unsubstituiert oder substituiert ist mit einem Substituenten ausgewählt aus der Gruppe bestehend aus F, Cl, Br, CF₃, Methyl, Methoxy, Hydroxy und NR(31)R(32);
- R(31) und R(32): unabhängig voneinander H oder Methyl;
- R30: Wasserstoff oder Alkyl mit 1, 2 oder 3 C-Atomen
- R7: Wasserstoff, Alkyl mit 1, 2, 3 oder 4 C-Atomen oder Phenyl, das unsubstituiert oder substituiert ist mit einem Substituenten ausgewählt aus der Gruppe bestehend aus F, Cl, Br, CF₃, Methyl, Methoxy, Hydroxy und NR(12)R(13);
- R(12) und R(13): unabhängig voneinander H oder Methyl;
oder
- R7: (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(14)R(15); R(14) und R(15)
unabhängig voneinander H oder Methyl;
oder
- R2 und R3: unabhängig voneinander Alkyl mit 1, 2, 3 oder 4 C-Atomen, Cycloalkyl mit 3, 4, 5, 6 oder 7 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(18)R(19);
- R(18) und R(19): unabhängig voneinander H oder Methyl;
oder
- R2 und R3: unabhängig voneinander (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(20)R(21);
- R(20) und R(21): unabhängig voneinander H oder Methyl;
oder
- R2 und R3: unabhängig voneinander SOₙ-R22;
- n: Null oder 2;
- R22: Alkyl mit 1, 2, 3 oder 4 C-Atomen, Cycloalkyl mit 3, 4, 5, 6 oder 7 C-Atomen oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(34)R(35);
- R(34) und R(35): unabhängig voneinander H oder Methyl;
- R⁴ und R⁵: unabhängig voneinander Wasserstoff, Alkyl mit 1, 2, 3 oder 4 C-Atomen, F, Cl, CF₃, -C≡N, -NO₂, SOₚ-R16, CO-R23 oder O-R24;
- p: Null oder 2;
- R23: Wasserstoff, Alkyl mit 1, 2, 3 oder 4 C-Atomen oder OR25; R25 Wasserstoff oder Alkyl mit 1, 2 oder 3 C-Atomen;
- R24: Wasserstoff, Alkyl mit 1, 2, 3 oder 4 C-Atomen oder Phenyl, das unsubstituiert oder substituiert ist mit einem Substituenten ausgewählt aus der Gruppe bestehend aus F, Cl, Br, CF₃, Methyl, Methoxy, Hydroxy und NR(28)R(29);
- R(28) und R(29): unabhängig voneinander H oder Methyl;
- R16: Alkyl mit 1, 2, 3 oder 4 C-Atomen oder Phenyl, das unsubstituiert oder substituiert ist mit einem Substituenten ausgewählt aus der Gruppe bestehend aus F, Cl, Br, CF₃, Methyl, Methoxy, Hydroxy und NR(26)R(27);
- R(26) und R(27): unabhängig voneinander H oder Methyl;
sowie deren physiologisch verträgliche Salze.

Ganz besonders bevorzugt sind Verbindungen der Formel I, in welcher bedeuten:
- R1: Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
oder
- R1: (C₁-C₉)-Heteroaryl,
das unsubstituiert oder substituiert ist mit einem Substituenten ausgewählt aus der Gruppe bestehend aus F, Cl, CF₃, CH₃ und Methoxy;
oder
- R1: (C₃-C₇)-Cycloalkyl;
- R2 und R3: unabhängig voneinander Wasserstoff, F, Cl, CF₃, -C≡N, CO-R6 oder O-R7;
- R6: Wasserstoff, Alkyl mit 1, 2, 3 oder 4 C-Atomen, OR30 oder Phenyl,
das unsubstituiert oder substituiert ist mit einem Substituenten ausgewählt aus der Gruppe bestehend aus F, Cl, CF₃, Methyl und Methoxy;
- R30: Wasserstoff, Methyl oder Ethyl;
- R7: Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
oder
- R7: (C₁-C₉)-Heteroaryl,
das unsubstituiert oder substituiert ist mit einem Substituenten ausgewählt aus der Gruppe bestehend aus F, Cl, Br, CF₃, CH₃ und Methoxy;
oder
- R2 und R3: unabhängig voneinander Alkyl mit 1, 2, 3 oder 4 C-Atomen, Cycloalkyl mit 3, 4, 5, 6 oder 7 C-Atomen oder Phenyl,
das unsubstituiert oder substituiert ist mit einem Substituenten ausgewählt aus der Gruppe bestehend aus F, Cl, CF₃, Methyl und Methoxy;
oder
- R2 und R3: unabhängig voneinander (C₁-C₉)-Heteroaryl,
das unsubstituiert oder substituiert ist mit einem Substituenten ausgewählt aus der Gruppe bestehend aus F, Cl, CF₃, CH₃ und Methoxy;
oder
- R2 und R3: unabhängig voneinander SOₙ-R22;
- n: Null oder 2;
- R22: Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, CF₃, Methyl und Methoxy;
- R4 und R5: unabhängig voneinander Wasserstoff, Methyl, F, Cl, CF₃, -C≡N, SO₂-R16, CO-R23 oder O-R24;
- R16: Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
- R23: Wasserstoff, Methyl oder OR25;
- R25: Wasserstoff, Methyl oder Ethyl;
- R24: Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
sowie deren physiologisch verträgliche Salze.

Bevorzugt sind des weiteren Verbindungen der Formel I in denen die Reste R1, R2, R3, R4 und R5 wie oben definiert sind und der Biphenylsubstituent wie in Formel Ia, Ib, Ic, Id, Ie, If, Ig oder Ih verknüpft ist 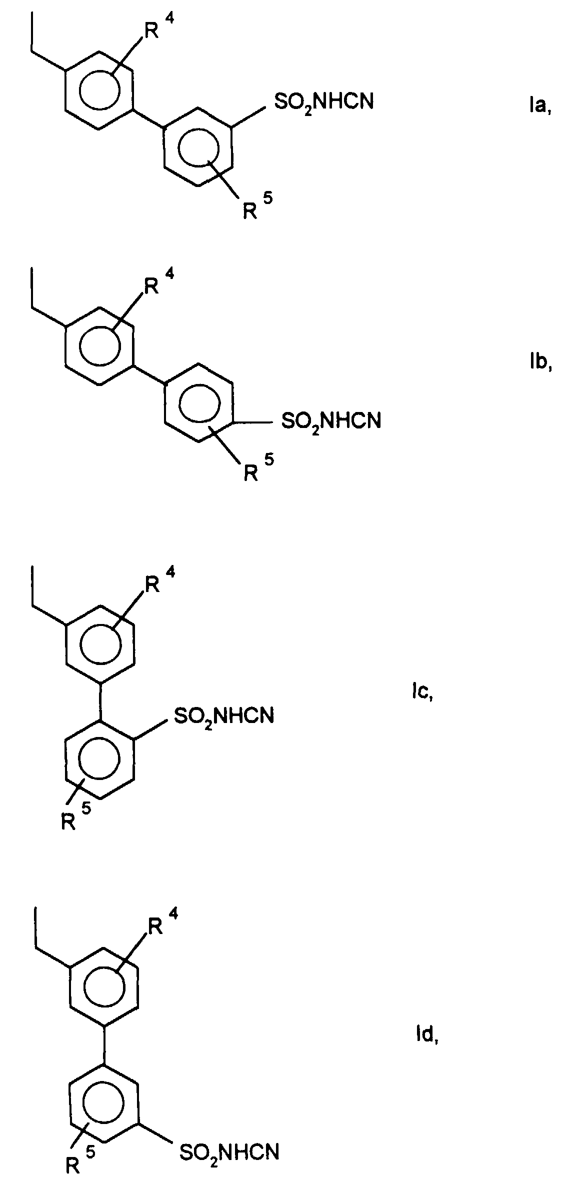 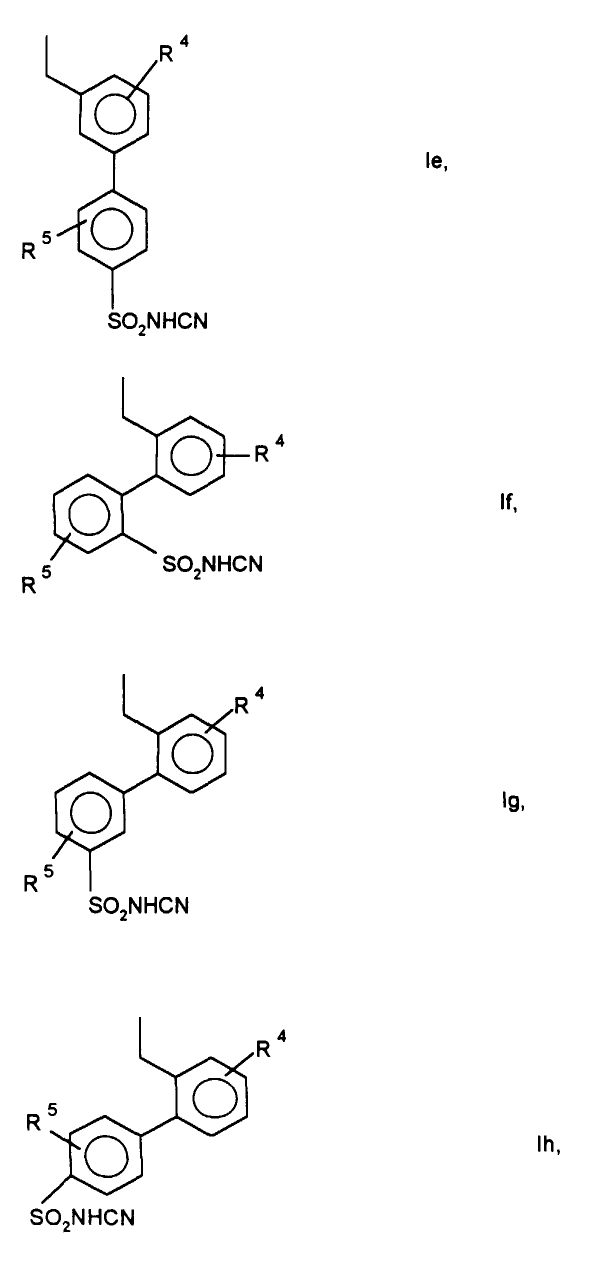 sowie deren physiologisch verträglichen Salze.

Bevorzugt sind desweiteren Verbindungen der Formel I, in denen die Reste R1, R2, R3, R4 und R5 wie oben definiert sind, wobei R⁴ und R⁵ nicht gleichzeitig Wasserstoff bedeuten.

Alkyl kann geradkettig oder verzweigt sein.

Unter Cycloalkyl werden auch Alkyl-substituierte Ringe verstanden. Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Gegebenenfalls vorkommende Stereozentren können sowohl (R)- als auch (S)-konfiguriert sein.

Unter physiologisch verträglichen Salzen von Verbindungen der Formel I versteht man sowohl deren organische als auch anorganische Salze, wie sie in Remington's Pharmaceutical Sciences [17. Auflage, Seite 1418 (1985)] beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die Erfindung betrifft auch ein Verfahren zur Herstellung der Verbindungen der Formel I, sowie deren physiologisch verträglicher Salze, das dadurch gekennzeichnet ist, daß man eine Verbindung der Formel II 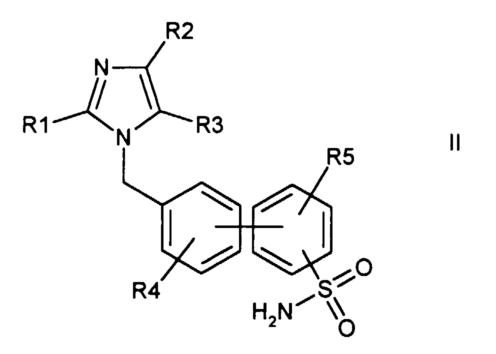 worin die Reste wie oben definiert sind und die entweder aus J. Med. Chem. 1995, *38*, 2357 bekannt sind oder analog hergestellt wurden, mit Bromcyan umsetzt. Die Reaktion erfolgt in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur von 0°C bis zu dem Siedepunkt des verwendeten Lösungsmittels in Frage, bevorzugt ist eine Temperatur von 60°C bis 120°C.

Die Erfindung betrifft weiterhin die Verwendung einer Verbindung der Formel 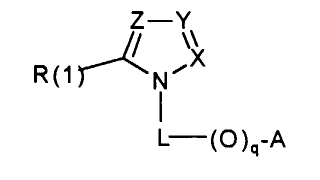 in welcher bedeuten:
a) X, Y und Z
   gleich oder verschieden, N oder CR(102)
b) R(1)
   1. (C₁-C₁₀)-Alkyl, 2. (C₃-C₁₀)-Alkenyl, 3. (C₃-C₁₀)-Alkinyl, 4. -OR(103), 5. (C₃-C₈)-Cycloalkyl, 6. (C₄-C₁₀)-Cycloalkylalkyl, 7. (C₅-C₁₀)-Cycloalkylalkenyl, 8. (C₅-C₁₀)-Cycloalkylalkinyl, 9. -(CH₂)ₘ-B-(CH₂)ₙ-R(104), 10. -Benzyl, 11. ein wie unter b) 1., 2., 3. oder 9. definierter Rest, der mit CO₂R(103) monosubstituiert ist, 12. ein wie unter b) 1., 2., 3. oder 9. definierter Rest, worin 1 bis alle H-Atome durch Fluor substituiert sind, oder 13. den unter b) 10. definierten Rest, der am Phenyl mit 1 oder 2 gleichen oder verschiedenen Resten aus der Reihe Halogen, (C₁-C₄)-Alkoxy und Nitro substituiert ist,
c) R(102)
   1. Wasserstoff, 2. Halogen, 3. Nitro, 4. CᵥF₂ᵥ₊₁, 5. Pentafluorphenyl, 6. Cyano, 7. -O-R(106), 8. Phenyl, 9. Phenyl-(C₁-C₃)-alkyl, 10. (C₁-C₁₀)-Alkyl, 11. (C₃-C₁₀)-Alkenyl, 12. Phenyl-(C₂-C₆)-Alkenyl, 13. 1-Imidazolyl-(CH₂)ₘ-, 14. 1,2,3-Triazolyl-(CH₂)ₙ-, 15. Tetrazolyl-(CH₂)ₘ-, 16. -(CH₂)ₒ₋₁-CHR(107)-OR(105), 17. -(CH₂)ₒ-O-CO-R(103), 18. - (CH₂)ₒ-S-R(106), 19. -S(O)ᵣ-R(119), 20. -CH=CH-(CH₂)ₘ-CHR(103)-OR(106), 21. -CH=CH-(CH₂)ₘ-CO-R(108), 22. -CO-R(108), 23. - CH=CH-(CH₂)ₘ-O-CO-R(107), 24. -(CH₂)ₘ-CH(CH₃)-CO-R(108), 25. - (CH₂)ₒ-CO-R(108), 26. -(CH₂)ₒ-O-[C=W]-NH-R(109), 27. -(CH₂)ₒ-NR(107)-[C=W]-OR(109), 28. -(CH₂)ₒ-NR(107)-CO-NHR(109), 29. - (CH₂)ₒ-NR(107)-SO₂R(109), 30. -(CH₂)ₒ-NR(107)-[C=W]-R(109), 31. - (CH₂)ₙF, 32. -(CH₂)ₙ-O-NO₂, 33. -CH₂-N₃, 34. -(CH₂)ₙ-NO₂, 35. - CH=N-NR(105)R(107), 36. Phthalimido-(CH₂)ₙ-,
   37. 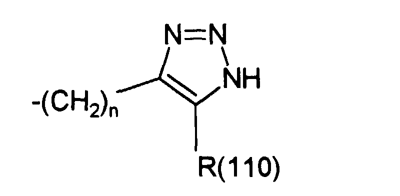
   38. 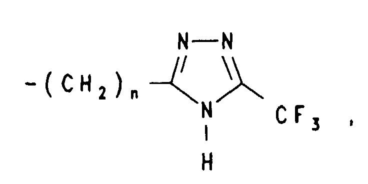
   39. 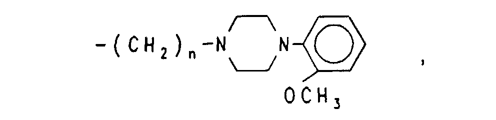
   40. 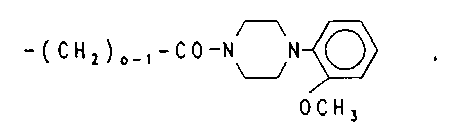
   41. 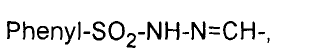
   42. 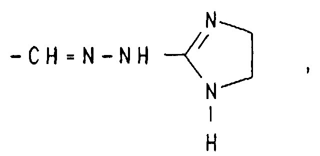
   43. -(CH₂)ₙ-SO₂-NR(107)-CS-NR(106)R(109)` 44. -(CH₂)ₙSO₂-NR(107)-CO-NR(106)R(109), 45. -(CH₂)ₒ-SO₂R(109), 46. einen wie unter c) 8. oder 9. definiertem Rest, der am Phenyl mit 1 oder 2 gleichen oder verschiedenen Resten aus der Reihe Halogen, Hydroxy, Methoxy, Trifluormethyl, CO₂R(103) und Phenyl substituiert ist, 47. einem wie unter c) 10., 11. oder 19. definierten Rest, worin ein bis alle H-Atome durch Fluor substituiert sind, 48. den unter c) 14. definierten Rest, der mit 1 oder 2 gleichen oder verschiedenen Resten aus der Reihe Methoxycarbonyl und (C₁-C₄)-Alkyl substituiert ist, 49. -(CH₂)ₙ-SO₂-NR(107)-CO-R(106), 50. -(CH₂)ₙ-SO₂-NR(107)CS-R(106),
d) R(103)
   1. Wasserstoff, 2. (C₁-C₈)-Alkyl, 3. (C₃-C₈)-Cycloalkyl, 4. Phenyl, 5. Benzyl, 6. den unter d) 2. definiertem Rest, worin 1 bis alle H-Atome durch Fluor substituiert sind;
e) R(104)
   1. Wasserstoff, 2. (C₁-C₆)-Alkyl, 3. (C₃-C₈)-Cycloalkyl, 4. (C₂-C₄)-Alkenyl oder 5. (C₂-C₄)-Alkinyl;
f) R(105)
   1. Wasserstoff, 2. (C₁-C₆)-Alkyl, 3. (C₃-C₈)-Cycloalkyl, 4. Phenyl oder 5. Benzyl;
g) R(106) und R(109)
   gleich oder verschieden 1. Wasserstoff, 2. (C₁-C₆)-Alkyl, das unsubstituiert ist oder substituiert mit 1 - 3 Substituenten ausgewählt aus der Gruppe bestehend aus (C₁-C₆)-Alkoxy, das seinerseits mit 1 - 3 Resten aus der Reihe Hydroxy, (C₁-C₆)-Alkoxy, Amino, Mono-(C₁-C₆)-Alkylamino, Di-(C₁-C₆)-Alkylamino substituiert sein kann, (C₂-C₁₀)-Alkenyl, Hydroxy, Amino, Mono-(C₁-C₆)-Alkylamino, Di-(C₁-C₆)-Alkylamino, (C₁-C₆)-Alkoxycarbonylamino, (C₆-C₁₂)-Aryl-(C₁-C₄)Alkoxycarbonylamino, (C₆-C₁₀)-Aryl, (C₆-C₁₀)Aryl-(C₁-C₃)-Alkyl, (C₁-C₉)-Heteroaryl, Carboxy und (C₁-C₄)-Alkoxycarbonyl; 3. (C₃-C₈)-Cycloalkyl,
      wobei der Cycloalkylteil unsubstituiert ist oder substituiert mit 1 - 3 Substituenten ausgewählt aus der Gruppe bestehend aus (C₁-C₄)-Alkyl und (C₂-C₄)-Alkenyl;
   4. (C₃-C₈)-Cycloalkyl-(C₁-C₃)-alkyl, 5. (C₆-C₁₂)-Aryl, vorzugweise Phenyl, 6. (C₆-C₁₀)-Aryl-(C₁-C₄)-Alkyl, 7. (C₁-C₉)-Heteroaryl,
      welches teilweise oder vollständig hydriert sein kann,
   8. einen wie unter g) 5., 6., 7., 9., 15., 16., 17., 19., 20. oder 21. definiertem Rest, substituiert mit 1 oder 2 gleichen oder verschiedenen Resten aus der Reihe Halogen, Hydroxy, (C₁-C₄)-Alkyl, Methoxy, Nitro, Cyano, CO₂R(103), Trifluormethyl, NR(111)R(112) und 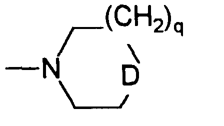
   9. (C₁-C₉)-Heteroaryl-(C₁-C₃)-alkyl,
      wobei der Heteroarylteil teilweise oder vollständig hydriert sein kann,
   10. (C₁-C₆)-Alkyl, worin 1 bis alle H-Atome durch Fluor substituiert sind, 11. (C₂-C₁₀)-Alkenyl, (C₂-C₁₀)-Alkenoyl oder (C₂-C₁₀)-Alkadienyl, 12. (C₃-C₈)-Cycloalkenyl, 13. (C₃-C₈)-Cycloalkenyl-(C₁-C₃)-Alkyl, 14. Bi- oder tricyclisches (C₄-C₁₀)-Cycloalkenyl-(C₁-C₄)-alkyl,
      das noch mit 1 - 3 (C₁-C₄)-Alkylresten substituiert sein kann;
   15. (C₆-C₁₀)-Aryl-(C₁-C₄)-Alkyl, 16. (C₆-C₁₀)-Aryl-(C₃-C₆)-Alkenyl, 17. (C₁-C₉)-Heteroaryl-(C₃-C₆)-Alkenyl, 18. (C₃-C₆)-Alkinyl, 19. (C₆-C₁₀)-Aryl-(C₃-C₆)-Alkinyl, 20. (C₁-C₉)-Heteroaryl-(C₃-C₆)-Alkinyl, 21. R(106) und R(109) gemeinsam mit dem sie tragenden N-Atom ein Heteroaryl,
      das auch teilweise oder vollständig hydriert sein kann;
h) R(107)
   1. Wasserstoff, 2. (C₁-C₆)-Alkyl, 3. (C₃-C₈)-Cycloalkyl, 4. (C₆-C₁₂)-Aryl-(C₁-C₆)-alkyl, vorzugsweise Benzyl, 5. Phenyl oder 6. (C₁ -C₉)-Heteroaryl;
i) R(108)
   1. Wasserstoff, 2. (C₁-C₆)-Alkyl, 3. (C₃-C₈)-Cycloalkyl, 4. Phenyl-(CH₂)_{q}-, 5. OR(106), 6. NR(111)R(112) oder 7. 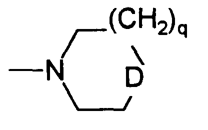
j) R(110)
   Cyano, Nitro oder CO₂R(107);
k) R(111) und R(112)
   gleich oder verschieden, 1. Wasserstoff, 2. (C₁-C₄)-Alkyl, 3. Phenyl, 4. Benzyl, oder 5. α-Methylbenzyl;
l) D NR(113), O oder CH₂;
m) R(113)
   Wasserstoff, (C₁-C₄)-Alkyl oder Phenyl;
n) A Biphenylyl,
   das unsubstituiert ist oder substituiert mit 1 - 4, vorzugsweise 1 bis 2 gleichen oder verschiedenen Substituenten R(114) oder R(115);
o) R(114)
   1. Halogen, 2. Nitroso, 3. Nitro, 4. Amino, 5. Cyano, 6. Hydroxy, 7. (C₁-C₆)-Alkyl, 8. (C₁-C₄)-Alkanoyl, 9. (C₁-C₄)-Alkanoyloxy, 10.CO₂R(103), 11. Methansulfonylamino, 12. Trifluormethansulfonylamino, 13. -CO-NH-OR(109), 14. -SO₂-NR(106)R(107), 15. -CH₂-OR(107), 16. (C₁-C₉)-Heteroaryl-(CH₂)_{q}-, vorzugsweise 1-Tetrazolyl, 17. (C₇-C₁₃)-Aroyl,
   18. 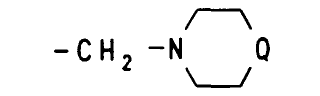
   19. 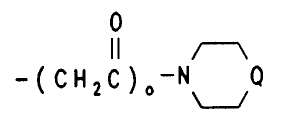 oder
   20. (C₆-C₁₂)-Aryl;
p) R(115)
   1. Wasserstoff, 2. (C₁-C₆)-Alkyl, 3. (C₃-C₈)-Cycloalkyl, 4. (C₆-C₁₂)-Aryl, 5. (C₇-C₁₃)-Aroyl, 6. (C₁-C₄)-Alkoxy, 7. (C₁-C₄)-Alkanoyloxy, 8. (C₁-C9)-Heteroaryl, 9. CO₂R(103), 10. Halogen, 11. Cyano, 12. Nitro, 13. NR(106)R(107), 14. Hydroxy, 15. -CO-NH-CHR(105)-CO₂R(103), 16. Sulfo, 17. -SO₃R(103), 18. -SO₂-NR(107)-CO-NR(106)R(109) oder -SO₂-NR(107)-CS-NR(106)R(109), 19. -NR(107)-CO-NR(106)-SO₂-CH₂-R(105), 20. -C(CF₃)₂OH, 21. Phosphonooxy, 22. -PO₃H₂, 23. -NH-PO(OH)₂, 24. -S(O)ᵣR(106), 25. -CO-R(108), 26. -CO-NR(106)R(109), 27. -CR(120)(OH)-PO(CH)₂, 28. den unter o) 20. definierten Rest,
   29. 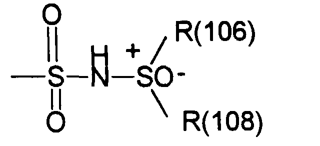
   30. 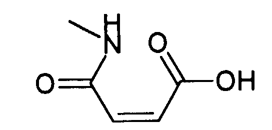
   31. 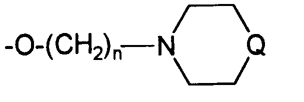
   32. 5-Tetrazolyl-NH-CO-, 33. -CO-NH-NH-SO₂-CF₃,
   34. 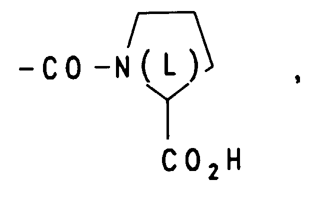
   35. 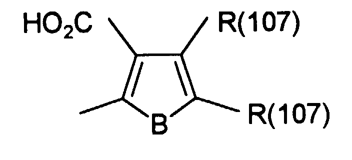
   36. 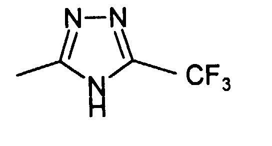
   37. 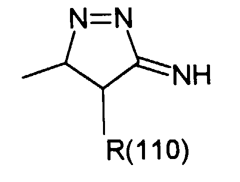
   38. 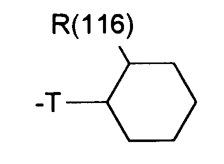
   39. 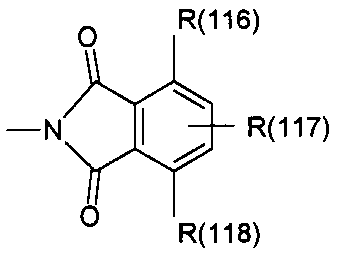
   40. -CO-NH-SO₂-R(119), 41. -SO₂-NH-CO-R(106) oder 42. den unter p) 4. definiertem Rest, substituiert mit 1 oder 2 gleichen oder verschiedenen Resten aus der Reihe Halogen, Cyano, Nitro, NR(106)R(107) und Hydroxy; 43. R(115) zusammen mit R(114) bedeutet -CO-NH-SO₂-, 44. -SO₂-NH-CO-O-R(106), 45. -SO₂-NH-SO₂-NR(106)R(109), 46. -SO₂-NH-SO₂-R(106);
q) B O, NR(107) oder S;
r) W O oder S;
s) L (C₁-C₃)-Alkandiyl;
t) R(116)
   CO₂R(103) oder CH₂CO₂R(103);
u) R(117)
   Wasserstoff, Halogen, (C₁-C₄)-Alkyl oder (C₁-C₄)-Alkoxy;
v) R(118)
   Wasserstoff, (C₁-C₄)-Alkyl oder Phenyl;
w) R(119)
   1. (C₁-C₆)-Alkyl, 2. (C₃-C₈)-Cycloalkyl, 3. Phenyl, 4. Benzyl oder 5. den unter w) 1. definiertem Rest, worin ein bis all H-Atome durch Fluor substituiert sind,
x) T
   1. eine Einfachbindung, 2. -CO-, 3. -CH₂-, 4. -O-, 5. -S-, 6. -NR(121)-, 7. -CO-NR(121), 8. -NR(121)-CO-, 9. -O-CH₂-, 10. -CH₂-O-, 11. -S-CH₂-, 12. -CH₂-S, 13. -NH-CR(120)R(122), 14. -NR(121)-SO₂, 15. SO₂-NR(121)-, 16. -CR(120)R(122)-NH, 17. -CH=CH-, 18. -CF=CF-, 19. -CH=CF-, 20. -CF=CH-, 21. -CH₂-CH₂-, 22. -CF₂-CF₂-, 23. -CH[OR(103)]-, 24. -CH(OCOR(105))-, 25. -C[N=R(123)]- oder 26. -[R(124)O]-C-[OR(125)]-
      y) R(120) und R(122)
         gleich oder verschieden, Wasserstoff, (C₁-C₅)-Alkyl, Phenyl, Allyl oder Benzyl;
      z) R(121)
         Wasserstoff, (C₁-C₆)-Alkyl, Benzyl oder Allyl;
      a') R(123)
         1. NR(120)R(121), 2. Ureido, 3. Thioureido, 4. Toluol-4-sulfonyl oder 5. Benzolsulfonylamino;
      b') R(124) und R(125)
         gleich oder verschieden, (C₁-C₄)-Alkyl oder gemeinsam -(CH₂)_{q}-;
      c') Q CH₂, NH, O oder S;
      d') m 1, 2, 3, 4 oder 5;
      e') n 1, 2, 3, 4 oder 5;
      f') o 1, 2, 3, 4, 5, 6, 7, 8, 9 oder 10;
      g') q Null oder 1;
      h') r Null, 1 oder 2;
      i') v 1, 2, 3, 4,5 oder bis 6;
sowie von deren physiologisch verträglichen Salze zur Herstellung eines Medikaments zur Behandlung oder Prophylaxe von durch ischämische Zustände hervorgerufenen Krankheiten, sowie zur Herstellung eines Medikaments zur Behandlung von gestörtem Atemantrieb.

Des weiteren betrifft die Erfindung die Verwendung einer Verbindung der Formel I zur Herstellung eines Medikaments zur Behandlung oder Prophylaxe von durch ischämische Zustände hervorgerufenen Krankheiten;
sowie die Verwendung einer Verbindung der Formel I zur Herstellung eines Medikaments zur Behandlung oder Prophylaxe des Herzinfarkts;
sowie die Verwendung einer Verbindung der Formel I zur Herstellung eines Medikaments zur Behandlung oder Prophylaxe der Angina Pectoris;
sowie die Verwendung einer Verbindung der Formel I zur Herstellung eines Medikaments zur Behandlung oder Prophylaxe von ischämischen Zuständen des Herzens;
sowie die Verwendung einer Verbindung der Formel I zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I zur Herstellung eines Medikaments zur Behandlung oder Prophylaxe von ischämischen Zuständen peripherer Organe und Gliedmaßen;
sowie die Verwendung einer Verbindung der Formel I zur Herstellung eines Medikaments zur Behandlung von Schockzuständen;
sowie die Verwendung einer Verbindung der Formel I zur Herstellung eines Medikaments zum Einsatz bei chirurgischen Operationen und Organtransplantationen;
sowie die Verwendung einer Verbindung der Formel I zur Herstellung eines Medikaments zur Konservierung und Lagerung von Transplantaten für chirurgische Maßnahmen;
sowie die Verwendung einer Verbindung der Formel I zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I zur Herstellung eines Medikaments zur Behandlung von gestörtem Atemantrieb;
sowie ein Arzneimittel, gekennzeichnet durch einen wirksamen Gehalt einer Verbindung der Formel I.

Den erfindungsgemäßen Verbindungen der Formel I ähnliche Verbindungen sind aus den US-Patentschriften 5,482,957 und 5,604,251 bekannt. Sie weisen jedoch nicht die nach der Erfindung stets vorhandene Sulfonylcyanamid-Seitenkette auf. Imidazolderivate als Angiotensin II-antagonisten werden auch in WO9523792, WO9523791, US 5391732, EP-A 648763 beschrieben. Derweiteren werden in US 5281614, Triazol-Derivate, in WO 9220662 und J. Med. Chem. (1994), 37 (17), 2808-2824 Triazolinon-Derivate als Angiotensin II-Rezeptorantagonisten beschrieben. Die bekannten Verbindungen sind Angiotensin II-Rezeptor-Antagonisten des Subtyps AT1, welche Wirkung bei den erfindungsgemäßen Verbindungen I nicht oder nur im geringen Ausmaß vorhanden ist.

Die erfindungsgemäßen Verbindungen der Formel I zeigen sehr gute antiarrhythmische Eigenschaften, wie sie beispielsweise für die Behandlung von Krankheiten wichtig sind, welche bei Sauerstoffmangelerscheinungen auftreten. Die Verbindungen I sind wegen ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.
Wegen ihrer schützenden Wirkungen gegen pathologische hypoxische und ischämische Situationen können die erfindungsgemäßen Verbindungen der Formel I infolge Inhibition des zellulären Na⁺-abhängigen Cl⁻/HCO₃⁻-Austauschmechanismus bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie schützen akut oder chronisch sauerstoffmangelversorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei Organ-Transplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen. Die Verbindungen der Formel I sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind. Darüber hinaus eignen sich die erfindungsgemäßen Verbindungen der Formel I ebenfalls zur Behandlung von Formen des Schocks, wie beispielsweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Es wurde gefunden, daß Inhibitoren des Na⁺-abhängigen Cl⁻/HCO₃⁻-Austauschers bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren.

Eine Verbesserung des Atemantriebes durch Stimulation der Chemorezeptoren mit Substanzen, die den Na⁺-abhängigen Cl⁻/HCO₃⁻-Austausch hemmen, führt zu einer Verbesserung der Atmung bei folgenden klinischen Zuständen und Krankheiten: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Arzneimittel, die eine Verbindung der Formel I enthalten, können dabei oral parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Die Verbindungen der Formel I können als einzige Wirkstoffe oder in Kombination mit anderen pharmakologisch wirksamen Verbindungen eingesetzt werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft desweiteren ganz allgemein die Kombination von a) NCBE-Inhibitoren und/oder deren physiologisch verträglichen Salze mit NHE-Inhibitoren und/oder deren physiologisch verträglichen Salze; b) NCBE-Inhibitoren und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze. Bevorzugt sind solche Kombinationen, in denen NCBE-Inhibitoren der allgemeinen Formel I und/oder deren physiologisch verträglichen Salze verwendet werden.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., "DIURETICS, Chemistry, Pharmacology and Medicine", J. WILEY & Sons (1983), 303 - 341 beschrieben sind oder um die in DE19737224.4 aufgeführten NHE-Inhibitoren.
Geeignete NHE-Inhibitoren sind beispielsweise auch Benzoylguanidine, wie sie in US 5292755, US 5373024, US 5364868, US 5591754, US 5516805, US 5559153, US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, DE 19624178.2, DE 19713427.0 beschrieben sind; ortho-substituierte Benzoylguanidine, wie sie in EP-A 556673, EP-A 791577, EP-A 794172, DE 19624178.2 beschrieben sind; ortho-amino-substituierte Benzoyl-guanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkyl- bzw. Alkenyl-carbonsäure- Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind Indenoylguanidine, wie sie in EP-A 738712 beschrieben sind; Benzyloxycarbonyl-guanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäure-diguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäureguanidine, wie sie in EP-A 790245 beschrieben sind; Bis-ortho-substituierte Benzoylguanidine, wie sie in DE 19621319.3 beschrieben sind; Substituierte 1-oder 2-Naphthylguanidine, wie sie in DE 19621482.3 und DE 19621483.1 beschrieben sind; Indanylidin-acetylguanidine, wie sie in EP 96112275.1 beschrieben sind; Phenylsubstituierte Alkenylcarbonsäure-guanidine, wie sie in DE 19633966.9 beschrieben sind; Aminopiperidyl-Benzoylguanidine, wie sie in EP 667341 beschrieben sind; Heterocycloxy-Benzylguanidine, wie sie in EP-A 694537 beschrieben sind; ortho-substituierte Benzoylguanidine, wie sie in EP704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie EP-A 708088 beschrieben sind; ortho-substituierte 5-Alkylsulfonyl-benzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, Polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyl- oder 3-Dihalophenyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonylbenzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoylguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie JP 9067332 beschrieben sind; Propenoylguanidine mit Indolsubstituenten, wie sie JP 9067340 beschrieben sind; oder Heteroarylsubstituierte Acryloylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am bevorzugtesten ist Cariporid oder dessen physiologisch verträgliches Salz.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridin-aktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.

Ein Beispiel eines solchen besonders vorteilhaften Kombinationspartners mit NCBE-Inhibitoren sind Blocker des nicht-inaktivierenden-Natriumkanals (Veratridinaktivierbarer Natriumkanal). Die Kombinationen eines NCBE-Inhibitors mit einem Blocker des nicht-inaktivierenden-Natriumkanals (Veratridin-aktivierbarer Natriumkanal) eignet sich zur Infarkt- und Re-infarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors mit einem Blocker des nicht-inaktivierenden-Natriumkanals infolge verstärkter Inhibierung des Na⁺ Einstroms in die Zelle als Arzneimittel zur Behandlung aller akuten oder chronischen, durch Ischämie ausgelösten Schäden oder dadurch primär oder sekundär induzierter Krankheiten verwendet werden. Dies betrifft ihre Verwendung als Arzneimittel für operative Eingriffe, z. B. bei Organtransplantationen, wobei die Kombinationen eines NCBE-Inhibitors mit einem Blocker des nicht-inaktivierendenNatriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors mit einem Blocker des nichtinaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NCBE-Inhibitors mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren mit NHE-Inhibitoren und/oder einer zusätzlichen aktiven Substanzen aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor und einen NHE-Inhibitor und/oder deren physiologisch verträglichen Salze; oder b) einen NCBE-Inhibitor und zusätzlich eine aktive Substanz aus einer anderen Herz-Kreislauf-Wirkstoffklasse und/oder deren physiologisch verträglichen Salze; oder c) einen NCBE-Inhibitor, einen NHE-Inhibitor und zusätzlich eine aktive Substanz aus einer anderen Herz-Kreislauf-Wirkstoffklasse, und/oder deren physiologisch verträglichen Salze.

Bevorzugt sind pharmazeutische Zubereitungen, die eine Verbindung der Formel I und/oder deren physiologisch verträgliches Salz als NCBE-Inhibitor enthalten.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor und einen NHE-Inhibitor und/oder deren physiologisch verträglichen Salze; oder b) einen NCBE-Inhibitor und zusätzlich eine aktive Substanz aus einer anderen Herz-Kreislauf-Wirkstoffklasse und/oder deren physiologisch verträglichen Salze; oder c) einen NCBE-Inhibitor, einen NHE-Inhibitor und zusätzlich eine aktive Substanz aus einer anderen Herz-Kreislauf-Wirkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen.
Bevorzugt sind Handelspackungen, die Verbindungen der Formel I als NCBE-Inhibitoren enthalten.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichstverhältnis vom NCBE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100, vorzugsweise 1:0,1 bis 1:10.

Die erfindungsgemäßen Kombination und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschieden Wirkstoffkomponenten im Berreich von 0,001 bis 100 mg/kg/Tag.

Liste der Abkürzungen:
- Bn: Benzyl
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- EI: electron impact
- eq: Äquivalent
- ES: Elektrospray-Ionisation
- Et: Ethyl
- FAB: Fast Atom Bombardment
- HEP: n-Heptan
- HOAc: Essigsäure
- Me: Methyl
- MeOH: Methanol
- mp: Schmelzpunkt
- MTB: Methyltertiärbutylether
- NCBE: Natriumabhängiger Chlorid/Bicarbonat-Austauscher
- NHE: Natrium/Wasserstoff-Austauscher
- NMP: N-Methylpyrrolidon
- RT: Raumtemperatur
- THF: Tetrahydrofuran
- TMU: N,N,N',N'-Tetramethylharnstoff
- Tol: Toluol
- ZNS: Zentralnervensystem

### Allgemeine Vorschrift zur Herstellung von Sulfonylcyanamiden aus Sulfonamiden

Das Sulfonamid-Ausgangsmaterial wird in 10 ml/mmol wasserfreiem Acetonitril gelöst, 3 Mol-Äquivalente K₂CO₃ sowie ein Mol-Äquivalent einer 5 N Lösung von BrCN in Acetonitril zugetropft und bis zum vollständigen Umsatz unter Rückfluß erhitzt (typische Reaktionszeit 1-6 Stunden). Das Reaktionsgemisch wird anschließend ohne weitere Aufarbeitung an Kieselgel chromatographiert.

### Beispiel 1: 2-Butyl-5-methylsulfanyl-3-(2'-cyanaminosulfonyl-biphenyl-4-ylmethyl)-3H-imidazol-4-carbonsäure-ethylester

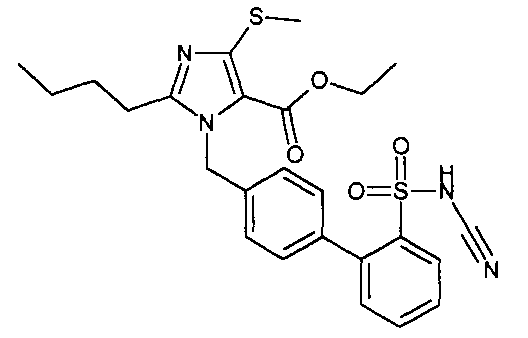

975 mg 1-[[2 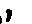 -(Aminosulfonyl)(1,1 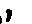 -biphenyl)-4-yl]methyl]-2-butyl-4-(methylthio)-1-H-imidazol-5-carbonsäure-ethylester (J. Med. Chem. 1995, 38, 2357) wurden in 10 ml wasserfreiem Acetonitril gelöst, dann wurden 276 mg K₂CO₃ und 2 ml einer 1 N Bromcyan-Lösung in Acetonitril zugegeben, und es wurde 4 h unter Rückfluß erhitzt. Das Reaktionsgemisch wurde an Kieselgel mit EE/MeOH 10 : 1 chromatographiert, und man erhielt 780 mg eines farblosen amorphen Feststoffs, beim Stehenlassen kristallisierend unter Zersetzung.
mp 111°C unter Zersetzung.
R_{f}(EE/MeOH 10:1) = 0.20 MS (FAB) : 513 (M+H)⁺

### Beispiel 2: 2-Butyl-5-methylsulfanyl-3-(3'-cyanaminosulfonyl-biphenyl-4-ylmethyl)-3H-imidazol-4-carbonsäure-ethylester

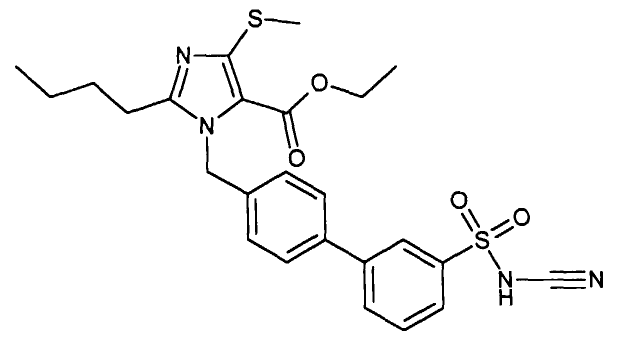

a) 3-Brom-N-dimethylaminomethylen-benzolsulfonamid
   20.3 g 3-Brom-benzolsulfonamid wurden in 120 ml DMF gelöst und bei RT 57 ml Dimethylformamid-dimethylacetal zugetropft. 5 h wurde bei RT gerührt und 3 Tage bei RT stehen gelassen. Das Reaktionsgemisch wurde dann auf 1,2 l Wasser gegossen, 90 Minuten nachgerührt und der weiße Niederschlag abfiltriert. Das Produkt wurde im Vakuum bei 50°C getrocknet, und man erhielt 20.5 g weißer Kristalle,
   mp 122°C.
   R_{f}(EE) = 0.59 MS (DCI) : 291 (M+H)⁺
b) 4'-Methyl-biphenyl-3-sulfonsäure-dimethylaminomethylenamid
   2.9 g 3-Brom-N-dimethylaminomethylen-benzolsulfonamid, 1.5 g p-Tolylboronsäure, 112 mg Pd(II)acetat und 224 mg Triphenylphosphin wurden in 60 ml Toluol sowie 17 ml Ethanol gelöst, 10 ml einer 2 N wäßrigen Na₂CO₃-Lösung zugegeben und 7 h unter Rückfluß gekocht. Das Reaktionsgemisch wurde in 150 ml einer gesättigten wäßrigen Na₂CO₃-Lösung und 150 ml Wasser aufgenommen und 3 mal mit je 200 ml EE extrahiert. Über Na₂SO₄ wurde getrocknet und das Solvens im Vakuum entfernt. Chromatographie an Kieselgel mit MTB lieferte 1.8 g eines farblosen Schaums.
   R_{f}(EE/HEP 2:1) = 0.22 MS (DCI) : 302 (M+H)⁺
c) 4'-Brommethyl-biphenyl-3-sulfonsäure-dimethylaminomethylenamid
   1.7 g 4'-Methyl-biphenyl-3-sulfonsäure-dimethylaminomethylenamid wurden in 30 ml Chlorbenzol gelöst und bei 130 ^{o}C 1.0 g N-Bromsuccinimid sowie 10 mg Benzoylperoxid zugegeben und 30 Minuten unter Rückfluß gekocht. Das Reaktionsgemisch wurde mit 150 ml EE aufgenommen und 2 mal mit je 150 ml eines 8:1-Gemisches aus gesättigter wäßriger Na₂CO₃- und gesättigter wäßriger Na₂SO₃-Lösung gewaschen. Die wäßrige Phase wurde anschließend noch 2 mal mit je 150 ml EE extrahiert. Die vereinigte organische Phase wurde über Na₂SO₄ getrocknet und das Solvens im Vakuum entfernt. Chromatographie an Kieselgel mit MTB lieferte 1.6 g eines blaßgelben Öls.
   R_{f}(EE) = 0.59 MS (DCI) : 291 (M+H)⁺
d) 2-Butyl-3-[3'-(dimethylaminomethylene-sulfamoyl)-biphenyl-4-ylmethyl]-5-( methylsulfanyl)- 3H-imidazol-4-carbonsäure-ethylester
   1.6 g 4'-Brommethyl-biphenyl-3-sulfonsäure-dimethylaminomethylenamid, 990 mg 2-Butyl-5-methylsulfanyl-3H-imidazol-4-carbonsäure-ethylester (J. Med. Chem. 1995, *38*, 2357) und 570 mg K₂CO₃ wurden in 12 ml DMF bei RT 4 h gerührt und über Nacht stehen gelassen. Anschließend wurde weitere 6 h bei RT gerührt, dann das Reaktionsgemisch in 125 ml einer gesättigten wäßrigen NaHCO₃-Lösung sowie 125 ml Wasser gegossen und 3 mal mit je 200 ml EE extrahiert. Über Na₂SO₄ wurde getrocknet und das Solvens im Vakuum entfernt. Chromatographie an Kieselgel mit EE/HEP 2:1 lieferte 1.1 g eines farblosen Öls.
   R_{f}(EE/HEP 1:1) = 0.10 MS (ES) : 543 (M+H)⁺
e) 2-Butyl-5-methylsulfanyl-3-(3'-sulfamoyl-biphenyl-4-ylmethyl)-3H-imidazol-4-carbonsäure-ethylester
   1.1 g 2-Butyl-3-[3'-(dimethylaminomethylene-sulfamoyl)-biphenyl-4-ylmethyl]-5-(methylsulfanyl)-3H-imidazol-4-carbonsäure-ethylester wurden in 20 ml Methanol gelöst und 10 ml einer gesättigten wäßrigen HCl-Lösung zugegeben. 4 h wurde unter Rückfluß erhitzt, dann mit einer wäßrigen 6 N NaOH-Lösung auf pH = 5-6 gestellt, mit 50 ml Wasser verdünnt und 3 mal mit je 150 ml EE extrahiert. Über Na₂SO₄ wurde getrocknet und das Solvens im Vakuum entfernt. Chromatographie an Kieselgel mit EE/HEP 1:1 lieferte 950 mg eines farblosen Schaums.
   R_{f}(EE/HEP 1:1) = 0.38 MS (ES) : 488 (M+H)⁺
f) 2-Butyl-5-methylsulfanyl-3-(3'-cyanaminosulfonyl-biphenyl-4-ylmethyl)-3H-imidazol-4-carbonsäure-ethylester
   720 mg 2-Butyl-5-methylsulfanyl-3-(3'-sulfamoyl-biphenyl-4-ylmethyl)-3H-imidazol-4-carbonsäure-ethylester wurden nach der allgemeinen Vorschrift zur Herstellung von Sulfonylcyanamiden aus Sulfonamiden umgesetzt, und man erhielt 550 mg eines amorphen Feststoffs.
   R_{f}(EE/MeOH 10:1) = 0.38 MS (ES) : 513 (M+H)⁺

Die Titelverbindungen der Beispiele 3 - 9 wurden analog Beispiel 2 synthetisiert:

### Beispiel 3: 2-Butyl-5-methylsulfanyl-3-(4'-cyanaminosulfonyl-biphenyl-4-ylmethyl)-3H-imidazol-4-carbonsäure-ethylester

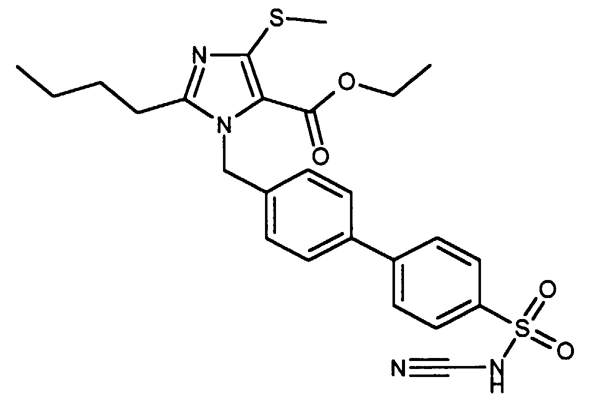

mp 244°C unter Zersetzung
R_{f}(EE/MeOH 10 : 1) = 0.17 MS (ES): 513 (M+H)⁺

### Beispiel 4: 2-Butyl-5-methylsulfanyl-3-(2'-cyanaminosulfonyl-biphenyl-3-ylmethyl)-3H-imidazol-4-carbonsäure-ethylester

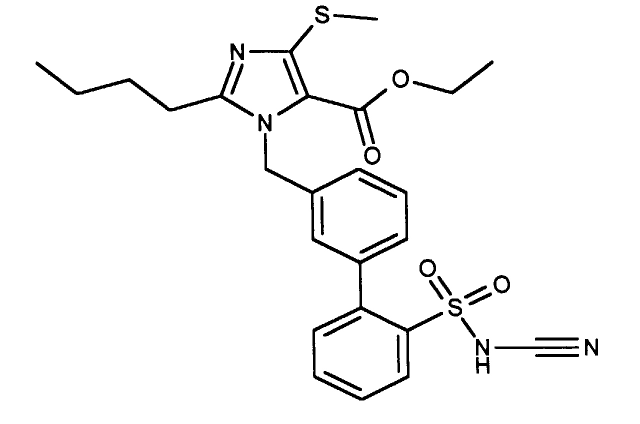

mp 118°C unter Zersetzung
R_{f}(EE/MeOH 10:1) = 0.19 MS (ES) : 513 (M+H)⁺

### Beispiel 5: 2-Butyl-5-methylsulfanyl-3-(3'-cyanaminosulfonyl-biphenyl-3-ylmethyl)-3H-imidazol-4-carbonsäure-ethylester

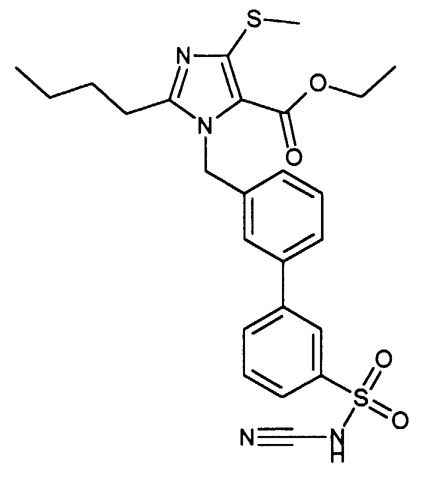

mp 112°C unter Zersetzung
R_{f}(EE/MeOH 10:1) = 0.15 MS (ES): 513 (M+H)⁺

### Beispiel 6: 2-Butyl-5-methylsulfanyl-3-(4'-cyanaminosulfonyl-biphenyl-3-ylmethyl)-3H-imidazol-4-carbonsäure-ethylester

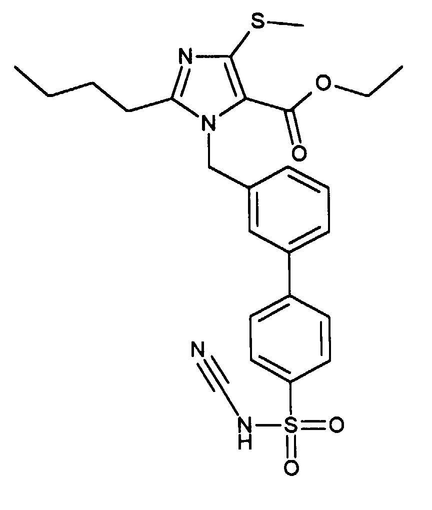

mp 120°C unter Zersetzung
R_{f}(EE/MeOH 10:1) = 0.36 MS (ES): 513 (M+H)⁺

### Beispiel 7: 2-Butyl-5-methylsulfanyl-3-(2'-cyanaminosulfonyl-biphenyl-2-ylmethyl)-3H-imidazol-4-carbonsäure-ethylester

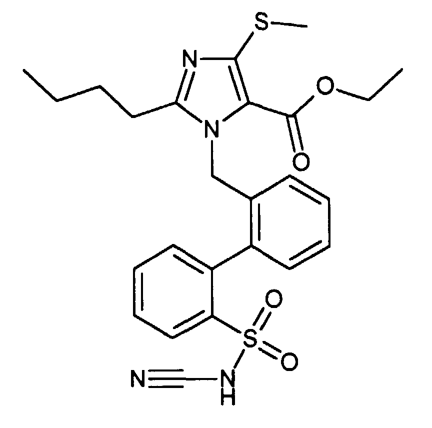

mp 105°C unter Zersetzung
R_{f}(EE/MeOH 10 : 1): = 0.23 MS (ES) : 513 (M+H)⁺

### Beispiel 8: 2-Butyl-5-methylsulfanyl-3-(3'-cyanaminosulfonyl-biphenyl-2-ylmethyl)-3H-imidazol-4-carbonsäure-ethylester

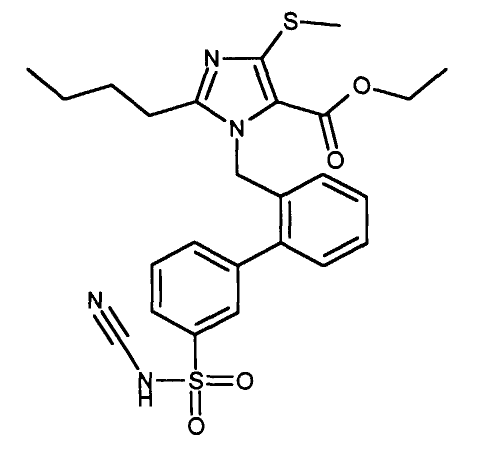

mp 108°C unter Zersetzung
R_{f}(EE/MeOH 10:1) = 0.27 MS (ES): 513 (M+H)⁺

### Beispiel 9: 2-Butyl-5-methylsulfanyl-3-(4'-cyanaminosulfonyl-biphenyl-2-ylmethyl)-3H-imidazol-4-carbonsäure-ethylester

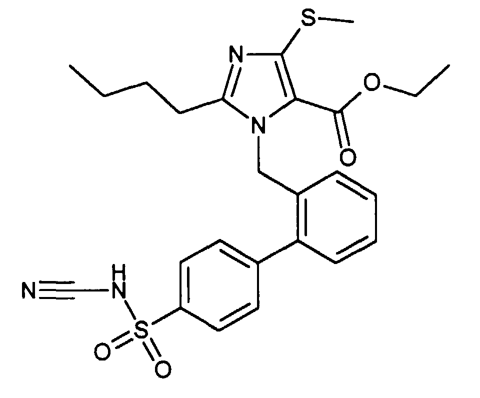

mp 116°C unter Zersetzung
R_{f}(EE/MeOH 10:1) = 0.23 MS (ES) : 513 (M+H)⁺

Die Titelverbindung des Beispiels 10 wurde analog Beispiel 2 aus 2-Cyclopropyl-5-methylsulfanyl-3-(4'-sulfamoyl-biphenyl-2-ylmethyl)-3H-imidazol-4-carbonsäure-ethylester (J. Med. Chem. 1995, 38, 2357) synthetisiert:

### Beispiel 10: 2-Cyclopropyl-5-methylsulfanyl-3-(2'-cyanaminosulfonyl-biphenyl-4-ylmethyl)-3H-imidazol-4-carbonsäure-ethylester

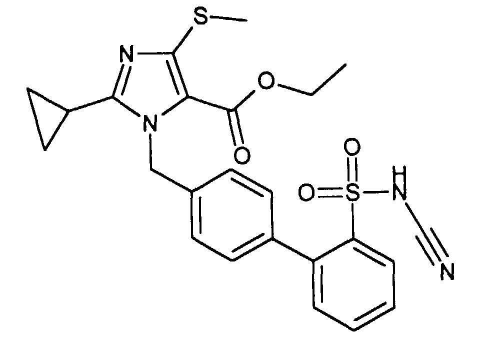

mp > 260°C
R_{f}(EE/MeOH 10:1) = 0.30 MS (ES) : 497 (M+H)⁺

### Beispiel 11: 4'-(4-Chloro-5-formyl-2-phenyl-imidazol-1-ylmethyl)-biphenyl-2-sulfonylcyanamid

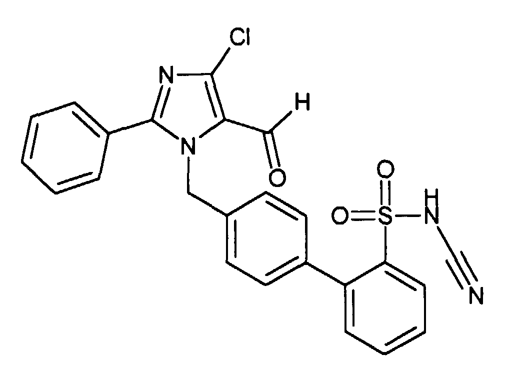

a) 4'-(4-Chloro-5-formyl-2-phenyl-imidazol-1-ylmethyl)-biphenyl-2-sulfonsäure-dimethylaminomethylenamid
   2.1 g 4-Chloro-5-formyl-2-phenyl-imidazol (Chem. Pharm. Bull. 1976, 24(5), 960), 3.8 g 4'-Brommethyl-biphenyl-2-sulfonsäure-dimethylaminomethylenamid (J. Med. Chem. 1995, 38, 2357) und 2.8 g K₂CO₃ wurden in 50 ml DMF 30 h bei RT gerührt. Das Reaktionsgemisch wurde zu 500 ml Wasser gegossen und 2 mal mit je 250 ml EE extrahiert. Die organische Phase wurde anschließend mit 100 ml Wasser gewaschen, über MgSO₄ getrocknet und das Solvens im Vakuum entfernt. Chromatographie an Kieselgel mit MTB lieferte 1.9 g eines weißen, kristallinen Pulvers, mp 193°C.
   R_{f}(MTB)=0.19 MS (ES) : 507 (M+H)⁺
b) 4'-(4-Chloro-5-formyl-2-phenyl-imidazol-1-ylmethyl)-biphenyl-2-sulfonamid 1.9 g 4'-(4-Chloro-5-formyl-2-phenyl-imidazol-1-ylmethyl)-biphenyl-2-sulfonsäure-dimethylaminomethylenamid wurden in 20 ml Ethanol gelöst und 20 ml einer gesättigten wäßrigen HCl-Lösung zugegeben. 2 Stunden lang wurde unter Rückfluß erhitzt, anschließend die flüchtigen Bestandteile im Vakuum entfernt, mit 200 ml Wasser aufgenommen, mit wäßriger NaOH-Lösung auf pH = 7 gestellt, 1 Stunde nachgerührt und der Niederschlag abgesaugt. Das Produkt wurde im Vakuum getrocknet, und man erhielt 1.7 g eines farblosen Feststoffs, mp 215°C unter Zersetzung.
   R_{f}(MTB) = 0.57 MS (FAB) : 452 (M+H)⁺
c) 4'-(4-Chloro-5-formyl-2-phenyl-imidazol-1-ylmethyl)-biphenyl-2-sulfonylcyanamid 800 mg 4'-(4-Chloro-5-formyl-2-phenyl-imidazol-1-ylmethyl)-biphenyl-2-sulfonamid wurden nach der allgemeinen Vorschrift zur Herstellung von Sulfonylcyanamiden aus Sulfonamiden umgesetzt, und man erhielt 650 mg eines amorphen Feststoffs.
   R_{f}(EE/MeOH 10:1) = 0.18 MS (ES) : 477 (M+H)⁺

### Beispiel 12: 4'-(4-Methoxy-5-formyl-2-phenyl-imidazol-1-ylmethyl)-biphenyl-2-sulfonylcyanamid

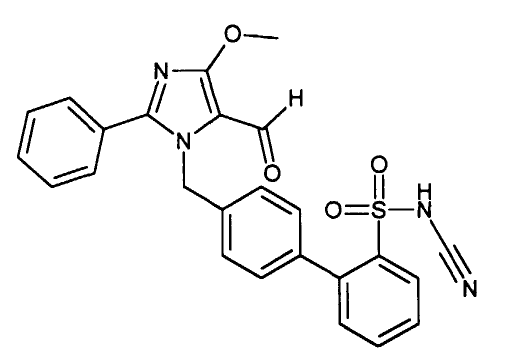

### a) 4'-(4-Methoxy-5-formyl-2-phenyl-imidazol-1-ylmethyl)-biphenyl-2-sulfonamid

700 mg 4'-(4-Chloro-5-formyl-2-phenyl-imidazol-1-ylmethyl)-biphenyl-2-sulfonamid (Beispiel 11 b) und 620 mg NaOH wurden in 10 ml Methanol gelöst und 1 Stunde unter Rückfluß erhitzt. Das Solvens wurde im Vakuum entfernt, mit 20 ml Wasser aufgenommen, mit wäßriger HCl-Lösung auf pH = 7 gestellt und der Niederschlag abfiltriert. Im Vakuum wurde getrocknet, und man erhielt 650 mg eines farblosen Feststoffs,
mp 188°C.
R_{f}(DIP/MTB 1:1) = 0.26 MS (FAB) : 448 (M+H)⁺

### b) 4'-(4-Methoxy-5-formyl-2-phenyl-imidazol-1-ylmethyl)-biphenyl-2-sulfonylcyanamid

650 mg 4'-(4-Methoxy-5-formyl-2-phenyl-imidazol-1-ylmethyl)-biphenyl-2-sulfonamid wurden nach der allgemeinen Vorschrift zur Herstellung von Sulfonylcyanamiden aus Sulfonamiden umgesetzt, und man erhielt 600 mg eines amorphen Feststoffs.
R_{f}(EE/MeOH 10:1) = 0.18 MS (ES) : 472 (M+H)+

### Beispiel 13: 2-Butyl-3-(2'-cyanaminosulfonylbiphenyl-4-ylmethyl)-3H-imidazol-4-carbonsäureethylester

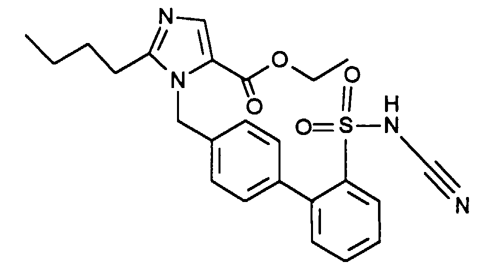

a) 1-[[2 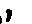 -(Aminosulfonyl)(1,1 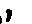 -biphenyl)-4-yl]methyl]-2-butyl-1-*H*-imidazol-5-carbonsäure-ethylester

244 mg 1-[[2 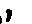 -(Aminosulfonyl)(1,1 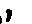 -biphenyl)-4-yl]methyl]-2-butyl-4-(methylthio)-1-*H*-imidazol-5-carbonsäure-ethylester (J. Med. Chem. **1995**, *38*, 2357) werden in 10 ml Ethanol gelöst und 200 mg Raney-Nickel zugegeben. Anschließend wird 6 h unter Rückfluß gekocht, weitere 200 mg Raney-Nickel zugegeben und erneut 3 h unter Rückfluß gekocht. Der Rückstand wird abfiltriert und das Solvens im Vakuum entfernt. Man erhält 200 mg eines farblosen Öls.
R_{f}(MTB/DIP 1:1) = 0.12 MS (FAB) : 442 (M+H)⁺

### b) 2-Butyl-3-(2'-cyanaminosulfonyl-biphenyl-4-ylmethyl)-3H-imidazol-4-carbonsäure-ethylester

140 mg 1-[[2 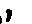 -(Aminosulfonyl)(1,1 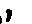 -biphenyl)-4-yl]methyl]-2-butyl-1-H-imidazol-5-carbonsäure-ethylester werden nach der allgemeinen Vorschrift zur Herstellung von Sulfonylcyanamiden aus Sulfonamiden umgesetzt (Reaktionszeit 1 h) und man erhält 90 mg weißer Kristalle, mp 121 °C unter Zersetzung.
R_{f}(EE/MeOH 5:1) = 0.20 IR (C≡N) : 2174.7 cm⁻¹ MS (ES) : 467 (M+H)⁺

### Beispiel 14: 4'-(2-Butyl-5-cyano-4-methoxy-imidazol-1-ylmethyl)-biphenyl-2-sulfonylcyanamid

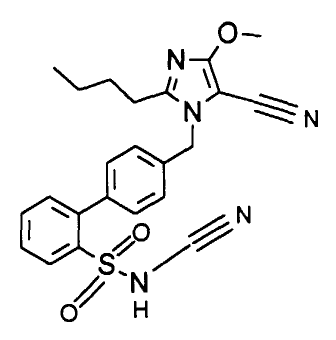

### a) 4'-[2-Butyl-5-(hydroxyimino-methyl)-4-methoxy-imidazol-1-ylmethyl]-biphenyl-2-sulfonamid

4.1 g 4'-(4-Methoxy-5-formyl-2-phenyl-imidazol-1-ylmethyl)-biphenyl-2-sulfonamid (Beispiel 12a) werden in 500 ml Methanol gelöst und 3.3 g Hydroxylamin-Hydrochlorid sowie 1.2 g 1,4-Diazabicyclo-[2,2,2]-octan zugegeben. 11 h wird bei RT gerührt, dann das Gemisch mit 200 ml einer gesättigten wäßrigen NaHCO₃-Lösung und 200 ml Wasser versetzt und 6 mal mit je 400 ml EE extrahiert. Über Na₂SO₄ wird getrocknet und die Solventien im Vakuum entfernt. Man erhält 4.5 g eines farblosen Öls.
R_{f}(MTB/Toluol 1:1) = 0.32

### b) 4'-(2-Butyl-5-cyano-4-methoxy-imidazol-1-ylmethyl)-biphenyl-2-sulfonsäure-acetyl-amid

210 mg 4'-[2-Butyl-5-(hydroxyimino-methyl)-4-methoxy-imidazol-1-ylmethyl]-biphenyl-2-sulfonamid werden in 4 ml Pyridin gelöst und 4 ml Acetanhydrid zugegeben. 170 Minuten wird unter Rückfluß gekocht, anschließend auf 200 ml einer eisgekühlten, gesättigten wäßrigen NaHCO₃-Lösung gegossen und 3 mal mit je 80 ml EE extrahiert. Über Na₂SO₄ wird getrocknet und das Solvens im Vakuum entfernt. Man erhält 230 mg eines farblosen Öls.
R_{f}(MTB/Toluol 1:1) = 0.14 MS (FAB) : 467 (M+H)⁺

### c) 4'-(2-Butyl-5-cyano-4-methoxy-imidazol-1-ylmethyl)-biphenyl-2-sulfonamid

2.1 g 4'-(2-Butyl-5-cyano-4-methoxy-imidazol-1-ylmethyl)-biphenyl-2-sulfonsäure-acetyl-amid werden in 40 ml einer 20% wäßrigen H₂SO₄-Lösung suspendiert und 3 h unter Rückfluß gekocht. Das Reaktionsgemisch wird in 400 ml einer 2/3 M Lösung von KH₂PO₄ in Wasser getropft und 3 mal mit je 300 ml EE extrahiert. Über Na₂SO₄ wird getrocknet und das Solvens im Vakuum entfernt. Chromatographie an Kieselgel mit MTB liefert 1.6 g eines farblosen Öls.
R_{f}(MTB) = 0.48

### d) 4'-(2-Butyl-5-cyano-4-methoxy-imidazol-1-ylmethyl)-biphenyl-2-sulfonylcyanamid

360 mg 4'-(2-Butyl-5-cyano-4-methoxy-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 1 h) und man erhält 300 mg weißer Kristalle mp 160 °C unter Zersetzung.
R_{f}(EE/MeOH 5:1) = 0.27 IR (C≡N) : 2177.2 cm⁻¹ MS (ES) : 450 (M+H)⁺

### Beispiel 15: 4'-(2-Butyl-4-chloro-5-formyl-imidazol-1-ylmethyl)-biphenyl-2-sulfonylcyanamide

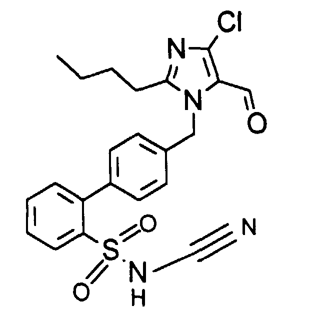

### a) 4'-(2-Butyl-4-chlor-5-formyl-imidazol-1-ylmethyl)-biphenyl-2-sulfonamid

10.6 g 4'-(2-Butyl-4-chlor-5-formyl-imidazol-1-ylmethyl)-biphenyl-2-sulfonsäure-dimethylaminomethyleneamid (J. Med. Chem. **1995**, *38*, 2357) werden in 200 ml Methanol gelöst und 100 ml einer gesättigten wäßrigen HCl-Lösung zugegeben. 2 h lang wird unter Rückfluß gekocht, nach Abkühlung mit 2 N wäßriger NaOH-Lösung auf pH = 5-6 gestellt und 4 mal mit je 200 ml EE extrahiert. Über Na₂SO₄ wird getrocknet und das Solvens im Vakuum entfernt. Man erhält 9.2 g eines farblolsen Öls.
R_{f}(EE/HEP 2:1) = 0.46

### b) 4'-(2-Butyl-4-chloro-5-formyl-imidazol-1-ylmethyl)-biphenyl-2-sulfonylcyanamide

160 mg 4'-(2-Butyl-4-chlor-5-formyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 75 Minuten) und man erhält 110 mg weißer Kristalle, mp 135 °C unter Zersetzung.
R_{f}(EE/MeOH 5:1) = 0.28 IR (C≡N) : 2176.9 cm⁻¹ MS (ESI) : 457 (M+H)⁺

Die Titelverbindungen der Beispiele 16-19 werden analog Beispiel 1 synthetisiert:

### Beispiel 16: 5-Methylsulfanyl-2-propyl-3-(2'-sulfamoyl-biphenyl-4-ylmethyl)-3H-imidazol-4-carbonsäure-ethylester

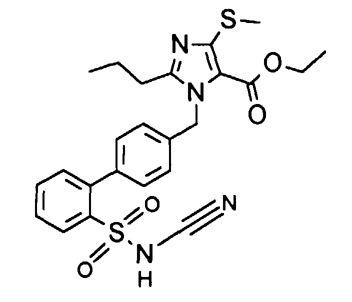

mp 165°C (Zersetzung) R_{f}(EE/MeOH 5:1) = 0.42 IR (C≡N) : 2178.6 cm⁻
¹MS (ES) : 499 (M+H)⁺

### Beispiel 17: 2-Ethyl-5-methylsulfanyl-3-(2'-cyanaminosulfonyl-biphenyl-4-ylmethyl)-3H-imidazole-4-carbonsäure-ethylester

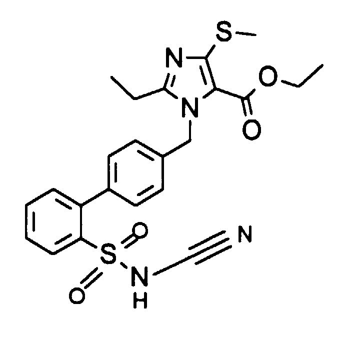

mp 94 °C (Zersetzung) R_{f}(EE/MeOH 5:1) = 0.38 IR (C≡N) : 2173.3 cm⁻
¹MS (ES) : 485 (M+H)⁺

### Beispiel 18: 2-Methyl-5-methylsulfanyl-3-(2'-cyanaminosulfonyl-biphenyl-4-ylmethyl)-3H-imidazole-4-carbonsäure-ethylester

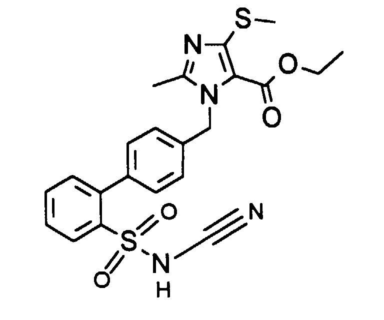

mp 169 °C (Zersetzung) R_{f}(EE/MeOH 5:1) = 0.29 IR (C≡N) : 2173.0 cm⁻
¹MS (ES) : 471 (M+H)⁺

### Beispiel 19: 2-Isopropyl-5-methylsulfanyl-3-(2'-cyanaminosulfonyl-biphenyl-4-ylmethyl)-3H-imidazole-4-carbonsäure-ethylester

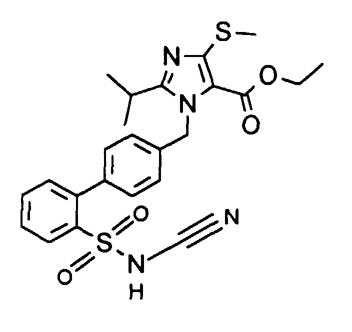

mp 276 °C (Zersetzung) R_{f}(EE/MeOH 5:1) = 0.28 IR (C≡N) : 2178.8 cm⁻
¹MS (ES) : 499 (M+H)⁺

### Beispiel 20: 2-Butyl-5-methylsulfanyl-3-(2'-cyanaminosulfonyl-biphenyl-4-ylmethyl)-3H-imidazol-4-carbonsäure-isopropylester

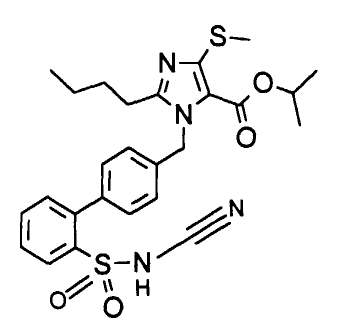

### a) 2-Butyl-5-methylsulfanyl-3-(2'-sulfamoyl-biphenyl-4-ylmethyl)-3H-imidazol-4-carbonsäure-isopropylester

980 mg 2-Butyl-5-methylsulfanyl-3-(2'-sulfamoyl-biphenyl-4-ylmethyl)-3H-imidazol-4-carbonsäure-ethylester werden in 10 ml Isopropanol gelöst und 590 µl Titan(IV)-isopropylat zugegeben. 9 h wird unter Rückfluß gekocht und anschließend weitere 590 µl Titan(IV)-isopropylat zugegeben. 8 h wird unter Rückfluß gekocht und erneut 1.2 ml Titan(IV)-isopropylat zugegeben. Nach weiteren 11 h Rückfluß wird auf 200 ml einer gesättigten wäßrigen NaHCO₃-Lösung gegossen und mit 200 ml Wasser verdünnt. 3 mal wird mit je 150 ml EE extrahiert, über Na₂SO₄ getrocknet und das Solvens im Vakuum entfernt. Chromatographie an Kieselgel mit EE/HEP 1:2 liefert 420 mg eines farblosen Öls.
R_{f}(MTB/HEP/CHCl₃ 2:1:1) = 0.36

### b) 2-Butyl-5-methylsulfanyl-3-(2'-cyanaminosulfonyl-biphenyl-4-ylmethyl)-3H-imidazol-4-carbonsäure-isopropylester

410 mg 2-Butyl-5-methylsulfanyl-3-(2'-sulfamoyl-biphenyl-4-ylmethyl)-3H-imidazol-4-carbonsäure-isopropylester werden nach der allgemeinen Vorschrift zur Herstellung von Sulfonylcyanamiden aus Sulfonamiden umgesetzt (Reaktionszeit 2 h) und man erhält 310 mg weißer Kristalle, mp 113 °C unter Zersetzung.
R_{f}(EE/MeOH 5:1) = 0.16 IR (C≡N) : 2178.2 cm⁻¹ MS (ES) : 527 (M+H)⁺

### Beispiel 21 2-Butyl-3-(2'-methyl-5'-cyanaminosulfonyl-biphenyl-4-ylmethyl)-5-methylsulfanyl-3H-imidazole-4-carbonsäure-ethylester

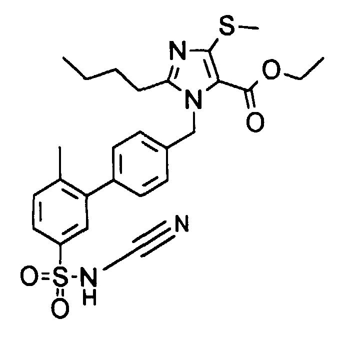

### a) 4,4,5,5-Tetramethyl-2-p-tolyl-[1,3,2]dioxaborolan

1.4 g p-Tolylboronsäure, 1.2 g Pinakol sowie eine katalytische Menge (etwa 2 mg) p-Toluolsulfonsäure werden 3 mal in je 40 ml Toluol suspendiert und die flüchtigen Bestandteile jeweils im Vakuum entfernt. Anschließend wird im Feinvakuum getrocknet und man erhält 2.0 g eines blaßgelben Öls.
R_{f}(EE/HEP 1:1) = 0.86

### b) 2-(4-Bromomethyl-phenyl)-4,4,5,5-tetramethyl-[1,3,2]dioxaborolan

2.0 g 4,4,5,5-Tetramethyl-2-p-tolyl-[1,3,2]dioxaborolan werden in 50 ml Chlorbenzol gelöst und bei Siedetemperatur portionsweise 1.7 g N-Bromsuccinimid und 5 mg Benzoylperoxid zugegeben. 4 h wird unter Rückfluß gekocht und nach dem Abkühlen mit 200 ml EE verdünnt. 2 mal wird mit je 100 ml einer 8:1 Mischung aus gesättigter wäßriger NaHCO₃-Lösung und gesättigter wäßriger Na₂SO₄-Lösung gewaschen, anschließend die wäßrige Phase noch 2 mal mit je 100 ml EE extrahiert und die vereinigten organischen Phasen über Na₂SO₄ getrocknet. Die Solventien werden im Vakuum entfernt und Chromatographie an Kieselgel mit EE/HEP 1:4 liefert 1.6 g eines hellbelben Öls.
R_{f}(EE/HEP 1:4) = 0.55 MS (DCI) : 297 (M+H)⁺

### c) 2-Butyl-5-methylsulfanyl-3-[4-(4,4,5,5-tetramethyl-[1,3,2]dioxaborolan-2-yl)-benzyl]-3H- imidazole-4-carbonsäure-ethylester

1.5 g 2-(4-Bromomethyl-phenyl)-4,4,5,5-tetramethyl-[1,3,2]dioxaborolan, 1.2 g 2-Butyl-5-methylsulfanyl-3H-imidazol-4-carbonsäure-ethylester (J. Med. Chem. **1995**, 38, 2357) und 700 mg K₂CO₃ werden in 15 ml DMF 14 h lang bei RT gerührt. Das Reaktionsgemisch wird auf 200 ml einer gesättigten wäßrigen NaHCO₃-Lösung gegossen, mit 200 ml Wasser verdünnt und 3 mal mit je 150 ml EE extrahiert. Über Na₂SO₄ wird getrocknet und das Solvens im Vakuum entfernt. Chromatographie an Kieselgel mit EE/HEP 1:4 liefert 1.4 g eines farblosen Öls.
R_{f}(EE/HEP 1:2) = 0.29 MS (FAB) : 459 (M+H)⁺

### d) 2-Butyl-5-methylsulfanyl-3-[4-(dihydroxyboryl)-benzyl]-3H-imidazol-4-carbonsäure-ethylester

1.2 g 2-Butyl-5-methylsulfanyl-3-[4-(4,4,5,5-tetramethyl-[1,3,2]dioxaborolan-2-yl)-benzyl]-3H- imidazole-4-carbonsäure-ethylester werden in 50 ml EE gelöst und 260 µl Diethanolamin zugegeben. 5 h wird bei RT gerührt, anschließend 4 h lang wird im Ultraschallreinigungsbad bei RT behandelt. Dann werden weitere 260 µl Diethanolamin zugegeben und weitere 2 h im Ultraschallreinigungsbad behandelt. Danach wird 14 h bei RT gerührt und der Niederschlag abfiltriert. Diese Fällung wird in 50 ml einer halbkonzentrierten wäßrigen NaHSO₄-Lösung aufgenommen und 2 h bei RT gerührt. Anschließend wird 3 mal mit je 150 ml EE extrahiert, über Na₂SO₄ getrocknet und das Solvens im Vakuum entfernt. Man erhält 390 mg eines amorphen Feststoffs.
R_{f}(EE) = 0.62 MS (FAB, +Glycerin) : 433 (M+56+H)⁺

### e) 3-Bromo-4-methyl-benzolsulfonamid

10 g 3-Brom-4-methyl-anilin werden in 22 ml Wasser suspendiert und 22 ml einer gesättigten wäßrigen HCl-Lösung zugetropft. 5 Minuten wird bei RT nachgerührt, anschließend auf -10 °C gekühlt und bei dieser Temperatur eine Lösung von 4.1 g NaNO₂ in 15 ml Wasser zugetropft. 45 Minuten wird bei -15 °C nachgerührt und portionsweise zu einer Suspension von 916 mg CuCl₂ x 2 H₂O und 92 mg CuCl in einer gesättigten Lösung von SO₂ in Eisessig bei RT zugegeben. Das Reaktionsgemisch wird auf dem Wasserbad langsam so lange erwärmt, bis die Stckstoffentwicklung beendet ist. Anschließend wird 3 mal mit je 250 ml Diethylether extrahiert, die organische Phase 2 mal mit je 80 ml Wasser gewaschen und über Na₂SO₄ getrocknet. Das Solvens wird im Vakuum entfernt, der Rückstand mit 75 ml Aceton aufgenommen, auf 0 °C abgekühlt und bei dieser Temperatur langsam eine gesättigte wäßrige NH₃-Lösung zugetropft, bis der pH = 10 erreicht. Dabei fällt ein Niederschlag aus, der abfiltriert und aus MTB umkristallisiert wird. Man erhält 2.5 g weißer Kristalle, mp 156 °C.
R_{f}(DIP) = 0.30 MS (DCI) : 250 (M+H)⁺

### f) 3-Bromo-N-dimethylaminomethylen-4-methyl-benzolsulfonamid

7.7 g 3-Bromo-4-methyl-benzolsulfonamid werden in 50 ml DMF gelöst und bei RT 20.5 ml Dimethylformamid-dimethylacetal zugetropft. 4 ½ h wird bei RT gerührt und anschließend das Produkt abgesaugt und im Vakuum getrocknet. Man erhält 9.0 g blaßgelber Kristalle, mp 162 °C.
R_{f}(EE) = 0.48 MS (ES) : 305 (M+H)⁺

### g) 2-Butyl-3-[5'-(dimethylaminomethylen-sulfamoyl)-2'-methyl-biphenyl-4-ylmethyl]-5-methylsulfanyl-3H-imidazole-4-carbonsäure-ethylester

390 mg 2-Butyl-5-methylsulfanyl-3-[4-(dihydroxyboryl)-benzyl]-3H-imidazol-4-carbonsäure-ethylester, 288 mg 3-Bromo-N-dimethylaminomethylen-4-methylbenzolsulfonamid, 11 mg Pd(II)-acetat und 28 mg Triphenylphosphin werden in 6 ml Toluol sowie 1.6 ml Ethanol suspendiert und 940 µl einer 2 M wäßrigen Na₂CO₃-Lösung zugegeben. Das Reaktionsgemisch wird 5 ¼ h unter Rückfluß gekocht, in 200 ml einer halbkonzentrierten wäßrigen NaHCO₃-Lösung gegossen und 3 mal mit je 150 ml EE extrahiert. Über Na₂SO₄ wird getrocknet und das Solvens im Vakuum entfernt. Chromatographie an Kieselgel liefert 150 mg eines farblosen Öls.
R_{f}(EE/HEP 2:1) = 0.19 MS (ES) : 557 (M+H)⁺

### h) 2-Butyl-3-[5'-sulfamoyl-2'-methyl-biphenyl-4-ylmethyl]-5-methylsulfanyl-3H-imidazol-4-carbonsäure-ethylester

140 mg 2-Butyl-3-[5'-(dimethylaminomethylen-sulfamoyl)-2'-methyl-biphenyl-4-ylmethyl]-5-methylsulfanyl-3H-imidazol-4-carbonsäure-ethylester werden in 2.5 ml Methanol gelöst und 1.3 ml einer gesättigten wäßrigen HCl-Lösung zugetropft. Das Reaktionsgemisch wird dann 75 Minuten unter Rückfluß gekocht, mit 6 N wäßriger NaOH-Lösung auf pH = 6 gestellt und mit 50 ml Wasser verdünnt. 5 mal wird mit je 50 ml EE extrahiert, über Na₂SO₄ getrocknet und das Solvens im Vakuum entfernt. Man erhält 110 mg eines amorphen Feststoffs.
R_{f}(EE/HEP 2:1) = 0.44 MS (ES) : 502 (M+H)⁺

### i) 2-Butyl-3-(2'-methyl-5'-cyanaminosulfonyl-biphenyl-4-ylmethyl)-5-methylsulfanyl-3H-imidazol-4-carbonsäure-ethylester

100 mg 2-Butyl-3-[5'-sulfamoyl-2'-methyl-biphenyl-4-ylmethyl]-5-methylsulfanyl-3H-imidazol-4-carbonsäure-ethylester werden nach der allgemeinen Vorschrift zur Herstellung von Sulfonylcyanamiden aus Sulfonamiden umgesetzt (Reaktionszeit 1 3/4 h) und man erhält 63 mg weißer Kristalle, mp 127 °C unter Zersetzung. R_{f}(EE/MeOH 5:1) = 0.09 IR (C≡N) : 2179.3 cm⁻¹ MS (ES) : 527 (M+H)⁺ Die Titelverbindungen der Beispiele 22-26 werden analog Beispiel 21 synthetisiert:

### Beispiel 22 2-Butyl-3-(2'-methyl-4'-cyanaminosulfonyl-biphenyl-4-ylmethyl)-5-methylsulfanyl-3H-imidazol-4-carbonsäure-ethylester

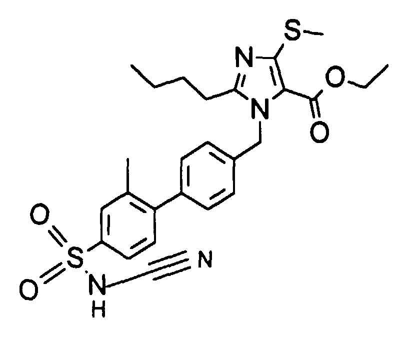

mp 211 °C R_{f}(EE/MeOH 5:1) = 0.20 IR (C≡N) : 2182.9 cm⁻¹
MS (ES) : 527 (M+H)⁺

### Beispiel 23: 2-Butyl-5-methylsulfanyl-3-(4'- cyanaminosulfonyl-2'-trifluoromethyl-biphenyl-4-ylmethyl)-3H-imidazol-4- carbonsäure-ethylester

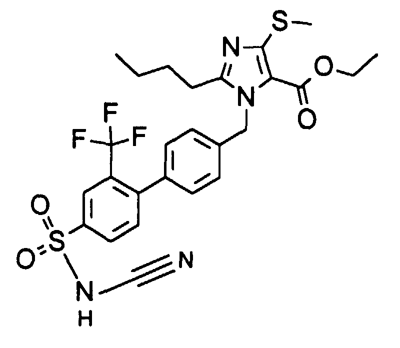

(amorph) R_{f}(EE/MeOH 10:1) = 0.20 IR (C≡N) : 2184.9 cm⁻¹
MS (ES) : 581 (M+H)⁺

### Beispiel 24: 2-Butyl-3-(2'-methyl-5'-cyanaminosulfonyl-biphenyl-3-ylmethyl)-5-methylsulfanyl-3H-imidazol-4-carbonsäure-ethylester

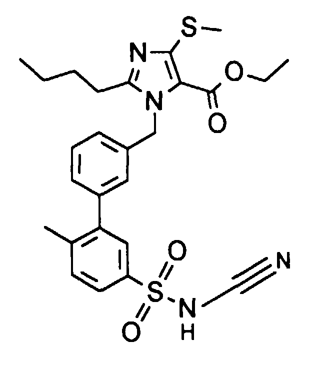

mp 160 °C (Zersetzung) R_{f}(EE/MeOH 10:1) = 0.18 IR (C≡N) : 2181.3 cm⁻
¹MS (ES) : 527 (M+H)⁺

### Beispiel 25: 2-Butyl-3-(2'-methyl-4'-cyanaminosulfonyl-biphenyl-3-ylmethyl)-5-methylsulfanyl-3H-imidazol-4-carbonsäure-ethylester

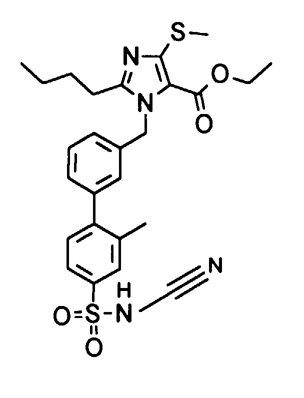

mp 115 °C (Zersetzung) R_{f}(EE/MeOH 10:1) = 0.18 IR (C≡N) : 2182.2 cm⁻
¹MS (ES) : 527 (M+H)⁺

### Beispiel 26: 2-Butyl-5-methylsulfanyl-3-(4'-cyanaminosulfonyl-2'-trifluoromethyl-biphenyl-3-ylmethyl)-3H-imidazol-4-carbonsäure-ethylester

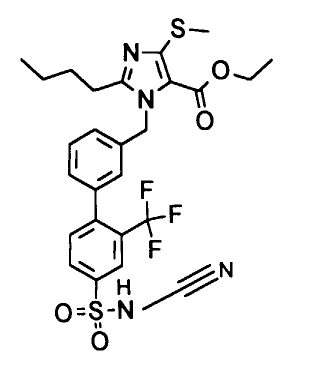

mp 122 °C (Zersetzung) R_{f}(EE/MeOH 10:1) = 0.27 IR (C≡N) : 2186.1 cm⁻
¹MS (ES) : 581 (M+H)⁺

### Beispiel 27: 4'-(2-Butyl-4-methylsulfanyl-imidazol-1-ylmethyl)-biphenyl-2-sulfonylcyanamid

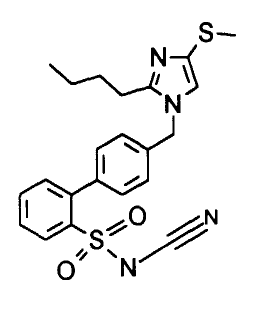

### a) 4'-(2-Butyl-4-methylsulfanyl-imidazol-1-ylmethyl)-biphenyl-2-sulfonamid

460 mg 2-Butyl-5-methylsulfanyl-3-(2'-sulfamoyl-biphenyl-4-ylmethyl)-3H-imidazol-4-carbonsäure werden in 5 ml Isopropanol gelöst, 220 µl Thionylchlorid zugespritzt und 4 ¾ h unter Rückfluß gekocht. Anschließend wird mit einer 6 N wäßrigen NaOH-Lösung auf pH = 6 gestellt, mit 100 ml Wasser verdünnt und 3 mal mit je 100 ml EE extrahiert. Über Na₂SO₄ wird getrocknet und das Solvens im Vakuum entfernt. Chromatographie an Kieselgel mit EE/HEP 1:1 liefert 300 mg eines farblosen Öls.
R_{f}(EE) = 0.58 MS (FAB) : 416 (M+H)⁺

### b) 4'-(2-Butyl-4-methylsulfanyl-imidazol-1-ylmethyl)-biphenyl-2-sulfonylcyanamid

260 mg 4'-(2-Butyl-4-methylsulfanyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h 20 Minuten) und man erhält 50 mg eines amorphen Pulvers.
R_{f}(EE/MeOH 5:1) = 0.19 IR (C≡N) : 2171.8 cm⁻¹ MS (ES) : 441 (M+H)⁺

### Beispiel 28: 4'-(4-Chloro-2-phenyl-imidazol-1-ylmethyl)-biphenyl-2-sulfonylcyanamid

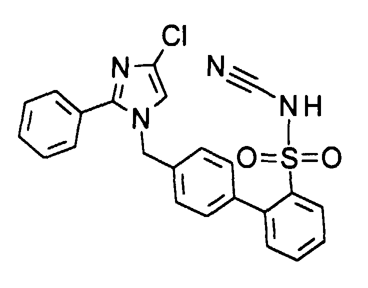

### a) 4'-(4-Chloro-2-phenyl-imidazol-1-ylmethyl)-biphenyl-2-sulfonamid

9.2 g 4'-(4-Chloro-5-formyl-2-phenyl-imidazol-1-ylmethyl)-biphenyl-2-sulfonsäure-dimethylaminomethylenamid (Beispiel 11 a), 95 ml einer gesättigten wäßrigen HCl-Lösung und 95 ml Ethanol werden 2 h unter Rückfluß gekocht. Nach dem Abkühlen wird mit 500 ml Wasser verdünnt, 2 mal mit je 500 ml EE extrahiert und die organische Phase 2 mal mit je 100 ml einer gesättigten wäßrigen NaCl- Lösung gewaschen. Über Na₂SO₄ wird getrocknet und das Solvens im Vakuum entfernt. Das Produktgemisch wird aus 200 ml EE umkristallisiert und man erhält 6.2 g 4'-(4-Chloro-5-formyl-2-phenyl-imidazol-1-ylmethyl)-biphenyl-2-sulfonamid (vergleiche Beispiel 11 b). Das Solvens der Mutterlauge wird im Vakuum entfernt und der Rückstand an Kieselgel mit MTB/DIP 1:1 chromatographiert. Man erhält 40 mg eines farblosen Öls.
R_{f} (MTB/DIP 1:1) = 0.22 MS (ES) : 424 (M+H)⁺

### b) 4'-(4-Chloro-2-phenyl-imidazol-1-ylmethyl)-biphenyl-2-sulfonylcyanamid

30 mg 4'-(4-Chloro-2-phenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 15 mg eines amorphen Pulvers.
R_{f}(EE/MeOH 10:1) = 0.09 MS (ES-) : 447 (M-H)⁻
ES- : Elektronenspray, negativer Modus

### Beispiel 29: 4'-(5-Acetyl-4-chloro-2-phenyl-imidazol-1-ylmethyl)-biphenyl-2-sulfonylcyanamid

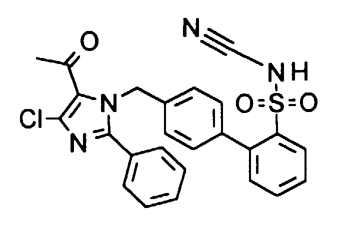

### a) 3-(4-Bromo-benzyl)-5-chloro-2-phenyl-3H-imidazol-4-carbaldehyd

3.0 g 4-Chloro-5-formyl-2-phenyl-imidazol (Chem. Pharm. Bull. **1976**, *24*(5), 960), 7.0 g 4-Brombenzylbromid und 11.7 g K₂CO₃ werden in 200 ml DMF 20 h lang bei RT gerührt. Anschließend wird das Reaktionsgemisch auf 500 ml Wasser gegossen, der Niederschlag abgesaugt und an Kieselgel mit DIP chromatographiert. Man erhält 3.9 g eines amorphen Schaums.
R_{f}(DIP) = 0.36 MS (ES): 375 (M+H)⁺

### b) 1-[3-(4-Bromo-benzyl)-5-chloro-2-phenyl-3H-imidazol-4-yl]-ethanol

3.8 g 3-(4-Bromo-benzyl)-5-chloro-2-phenyl-3H-imidazol-4-carbaldehyd werden in 50 ml THF gelöst und eine Grignard-Lösung, hergestellt aus 385 mg Magnesium-Spänen und 990 µl Methyliodid in 50 ml Diethylether, bei RT langsam zugespritzt. 3 Tage wird bei RT gerührt, anschließend 200 ml einer 5% wäßrigen NaHSO₄-Lösung zugegeben und 2 mal mit je 200 ml EE extrahiert. Über Na₂SO₄ wird getrocknet und das Solvens im Vakuum entfernt. Man erhält 3.7 g eines farblosen Öls.
R_{f}(DIP) = 0.13 MS (DCI) : 391 (M+H)⁺

### c) 1-[3-(4-Bromo-benzyl)-5-chloro-2-phenyl-3H-imidazol-4-yl]-ethanon

3.7 g 1-[3-(4-Bromo-benzyl)-5-chloro-2-phenyl-3H-imidazol-4-yl]-ethanol wird in 20 ml Essigsäure gelöst und bei 15 °C mit einer Lösung von 15.6 g (NH₄)₂Ce(NO₃)₆ in 30 ml Wasser langsam versetzt. Bei 10 °C wird 30 Minuten lang nachgerührt, dann läßt man auf RT aufwärmen. Es wird mit 200 ml Wasser verdünnt, mit NaHCO₃ auf pH = 5 gestellt und das Produkt abgesaugt. Chromatographie je einmal mit DIP und MTB liefert 2.0 g eines farblosen Öls.
R_{f}(DIP) = 0.42 MS (DCI) : 389 (M+H)⁺

### d) 4'-(5-Acetyl-4-chloro-2-phenyl-imidazol-1-ylmethyl)-biphenyl-2-sulfonsäure-tert-butylamid

21.0 g 1-[3-(4-Bromo-benzyl)-5-chloro-2-phenyl-3H-imidazol-4-yl]-ethanon, 2.0 g N-tert-Butyl-2-dihydroxyboran-2-yl-benzolsulfonamid, 135 mg Triphenylphosphin, 58 mg Pd(II)-acetat und 1.1 g Na₂CO₃ werden in 50 ml Toluol und 10 ml Wasser 6 h unter Rückfluß gekocht. Das Gemisch wird mit 200 ml EE verdünnt, 2 mal mit je 50 ml einer gesättigten wäßrigen NaHCO₃-Lösung gewaschen, über Na₂SO₄ getrocknet und die Solventien im Vakuum entfernt. Chromatographie an Kieselgel mit DIP liefert 1.4 g eines farblosen Öls.
MS (ES) : 522 (M+H)⁺

### e) 4'-(5-Acetyl-4-chloro-2-phenyl-imidazol-1-ylmethyl)-biphenyl-2-sulfonamid

1.0 g 4'-(5-Acetyl-4-chloro-2-phenyl-imidazol-1-ylmethyl)-biphenyl-2-sulfonsäure-tert-butylamid und 230 µl Anisol werden in 5 ml Trifluoressigsäure gelöst und 24 h bei RT stehen gelassen. Die flüchtigen Bestandteile werden anschließend im Vakuum entfernt und der Rückstand mit 50 ml Heptan digeriert. Man erhält 870 mg eines amorphen Feststoffs.
R_{f}(MTB) = 0.66 MS (ES) : 466 (M+H)⁺

### f) 4'-(5-Acetyl-4-chloro-2-phenyl-imidazol-1-ylmethyl)-biphenyl-2-sulfonylcyanamid

100 mg 4'-(5-Acetyl-4-chloro-2-phenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 30 mg eines amorphen Pulvers.
R_{f}(EE/MeOH 10:1) = 0.20 IR (C≡N) : 2182.0 cm⁻¹ MS (ES-) : 489 (M-H)⁻

### Beispiel 30: 4'-(5-Acetyl-4-methoxy-2-phenyl-imidazol-1-ylmethyl)-biphenyl-2-sulfonylcyanamid

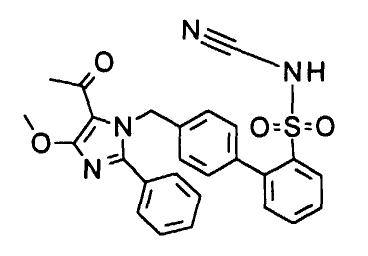

### a) 4'-(5-Acetyl-4-methoxy-2-phenyl-imidazol-1-ylmethyl)-biphenyl-2-sulfonamid

760 mg 4'-(5-Acetyl-4-chloro-2-phenyl-imidazol-1-ylmethyl)-biphenyl-2-sulfonamid und 650 mg NaOH werden in 10 ml Methanol 22 h unter Rückfluß gekocht. Das Solvens wird anschließend im Vakuum entfernt, in 20 ml Wasser wird der Rückstand aufgenommen, mit einer 10% wäßrigen HCl-Lösung auf pH = 6 gestellt und 3 mal mit je 50 ml EE extrahiert. Über Na₂SO₄ wird getrocknet und das Solvens im Vakuum entfernt. Chromatographie an Kieselgel mit MTB/DIP 1:1 liefert 230 mg eines viskosen Öls.
R_{f}(MTB/DIP 1:1) = 0.31 MS (ES) : 462 (M+H)⁺

### b) 4'-(5-Acetyl-4-methoxy-2-phenyl-imidazol-1-ylmethyl)-biphenyl-2-sulfonylcyanamid

210 mg 4'-(5-Acetyl-4-methoxy-2-phenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 181 mg eines amorphen Pulvers.
R_{f}(EE/MeOH 10:1) = 0.14 IR (C≡N) : 2179.7 cm⁻¹ MS (ES-) : 485 (M-H)⁻

### Beispiel 31:

### 4'-(2,4,5-Triphenyl-imidazol-1-ylmethyl)-biphenyl-2-sulfonylcyanamid

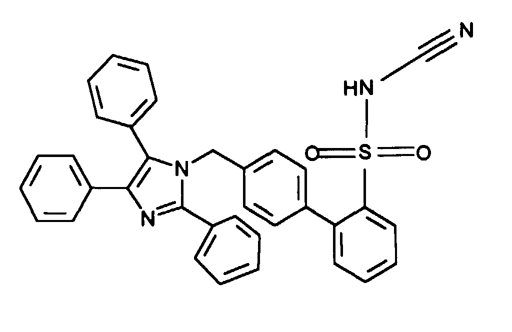

### a) 4'-(2,4,5-Triphenyl-imidazol-1-ylmethyl)-biphenyl-2-sulfonsäure-dimethylaminomethyleneamid

1.5 g 2,4,5-Triphenyl-imidazol, 1.9 g 4'-Bromomethyl-biphenyl-2-sulfonsäure-dimethylaminomethyleneamid (J. Med. Chem. **1995**, *38*, 2357) und 2.1 g K₂CO₃ werden in 50 ml DMF 6 Tage bei RT gerührt. Das Reaktionsgemisch wird auf 300 ml Wasser gegossen und mit 500 ml EE extrahiert. Die organische Phase wird 3 mal mit je 250 ml einer gesättigten wäßrigen NaCl-Lösung gewaschen, über Na₂SO₄ getrocknet und das Solvens im Vakuum entfernt. Man erhält 2.8 g eines viskosen Öls.
MS (ES) : 597 (M+H)⁺

### b) 4'-(2,4,5-Triphenyl-imidazol-1-ylmethyl)-biphenyl-2-sulfonamid

1.9 g 4'-(2,4,5-Triphenyl-imidazol-1-ylmethyl)-biphenyl-2-sulfonsäure-dimethylaminomethyleneamid werden in 20 ml Ethanol und 20 ml einer gesättigten wäßrigen HCl-Lösung 3 h unter Rückfluß gekocht. Die flüchtigen Bestandteile werden im Vakuum entfernt, mit 100 ml Wasser aufgerührt und das Produkt abfiltriert. Man erhält 1.0 g eines amorphen Pulvers.
R_{f}(MTB) = 0.47 MS (ES) : 542 (M+H)⁺

### c) 4'-(2,4,5-Triphenyl-imidazol-1-ylmethyl)-biphenyl-2-sulfonylcyanamid

1.0 g 4'-(2,4,5-Triphenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 970 mg eines amorphen Pulvers.
R_{f}(EE/MeOH 10:1) = 0.26 IR (C≡N) : 2173.5 cm⁻¹ MS (FAB) : 567 (M+H)⁺

### Beispiel 32: 3'-Chloro-4'-(4-chloro-5-formyl-2-phenyl-imidazol-1-ylmethyl)-biphenyl-2-sulfonylcyanamid

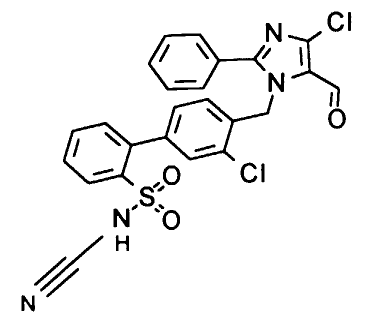

### a) 4-Brom-1-brommethyl-2-chlor-benzol

7.1 ml 4-Brom-2-chlortoluol werden in 20 ml Chlorbenzol gelöst und bei 130 °C portionsweise mit einer Mischung aus 9.4 g N-Bromsuccinimid und 200 mg Dibenzoylperoxid versetzt. 30 Minuten wird unter Rückfluß gekocht, nach dem Abkühlen mit 100 ml CH₂Cl₂ verdünnt und je 1 mal mit 50 ml einer gesättigten wäßrigen Na₂SO₃-Lösung und 100 ml einer gesättigten wäßrigen NaHCO₃-Lösung gewaschen.Über Na₂SO₄ wird getrocknet und das Solvens im Vakuum entfernt. Man erhält 11.0 g eines blaßgelben Öls.
R_{f}(EE/HEP 1:8) = 0.49 MS (DCI) : 283 (M+H)⁺

### b) 3-(4-Brom-2-chlor-benzyl)-5-chlor-2-phenyl-3H-imidazol-4-carbaldehyd

1.5 g 4-Chloro-5-formyl-2-phenyl-imidazol (Chem. Pharm. Bull. **1976**, *24*(5), 960), 5.8 g K₂CO₃ und 8.0 g 4-Brom-1-brommethyl-2-chlor-benzol werden in 50 ml DMF 24 h bei RT gerührt. Anschließend wird mit 250 ml EE verdünnt und 2 mal mit je 100 ml Wasser und 1 mal mit 100 ml einer gesättigten wäßrigen NaCl-Lösung gewaschen. Über Na₂SO₄ wird getrocknet und die Solventien im Vakuum entfernt. Chromatographie an Kieselgel mit EE/HEP 1:4 liefert 2.2 g eines farblosen Öls.
R_{f}(EE/HEP 1:4) = 0.47 MS(ES) : 411 (M+H)⁺

### c) 3'-Chloro-4'-(4-chloro-5-formyl-2-phenyl-imidazol-1-ylmethyl)-biphenyl-2-sulfonsäure-tert-butylamid

2.2 g 3-(4-Brom-2-chlor-benzyl)-5-chlor-2-phenyl-3H-imidazol-4-carbaldehyd, 2.0 g N-tert-Butyl-2-dihydroxyboran-2-yl-benzolsulfonamid (*J*. *Med*. *Chem*. **1997**, *40*, 547), 140 mg Triphenylphosphin, 64 mg Pd(II)-acetat sowie 1.2 g Na₂CO₃ werden in 30 ml Toluol, 10 ml Ethanol sowie 10 ml Wasser gelöst und 3 h unter Rückfluß gekocht. Nach dem Abkühlen werden 200 ml einer gesättigten wäßrigen NaHCO₃-Lösung zugegeben und 3 mal mit je 200 ml EE extrahiert. Über MgSO₄ wird getrocknet und das Solvens im Vakuum entfernt. Chromatographie an Kieselgel liefert 1.5 g eines farblosen Schaums.
R_{f}(DIP) = 0.25 MS (ES) : 542 (M+H)⁺

### d) 3'-Chloro-4'-(4-chloro-5-formyl-2-phenyl-imidazol-1-ylmethyl)-biphenyl-2-sulfonamid

1.5 g 3'-Chloro-4'-(4-chloro-5-formyl-2-phenyl-imidazol-1-ylmethyl)-biphenyl-2-sulfonsäure-tert-butylamid und 340 µl Anisol werden in 10 ml Trifluoressigsäure gelöst und bei RT 24 h gerührt. Die flüchtigen Bestandteile werden im Vakuum entfernt, je 2 mal mit 50 ml Toluol aufgenommen und erneut die flüchtigen Bestandteile im Vakuum entfernt. Man erhält 1.5 g eines farblosen Schaums.
R_{f}(DIP)=0.15 MS (ES) : 486 (M+H)⁺

### e) 3'-Chloro-4'-(4-chloro-5-formyl-2-phenyl-imidazol-1-ylmethyl)-biphenyl-2-sulfonylcyanamid

400 mg 3'-Chloro-4'-(4-chloro-5-formyl-2-phenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 970 mg eines amorphen Pulvers.
R_{f}(EE/MeOH 10:1) = 0.23 IR (C≡N) : 2179.2 cm⁻¹ MS (FAB) : 511 (M+H)⁺

Die Titelverbindung des Beispiels 33 wurde analog Beispiel 32 synthetisiert:

### Beispiel 33: 3'-Fluoro-4'-(4-chloro-5-formyl-2-phenyl-imidazol-1-ylmethyl)-biphenyl-2-sulfonylcyanamid

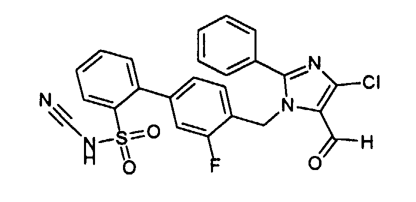

R_{f}(EE/MeOH 10:1) = 0.28 IR (C≡N) : 2177.7 cm⁻¹ MS (ES-) : 493 (M-H)⁻

Die Titelverbindung des Beispiels 34 wurde analog Beispiel 12 aus 3'-Chloro-4'-(4-chloro-5-formyl-2-phenyl-imidazol-1-ylmethyl)-biphenyl-2-sulfonamid (Beispiel 32 d) synthetisiert:

### Beispiel 34: 3'-Chloro-4'-(4-methoxy-5-formyl-2-phenyl-imidazol-1-ylmethyl)-biphenyl-2-sulfonylcyanamid

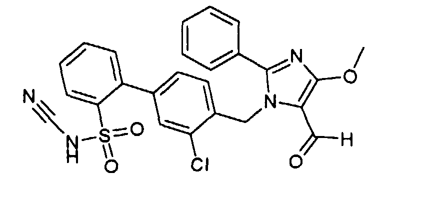

R_{f}(EE/MeOH 10:1) = 0.26 IR (C≡N) : 2177.4 cm⁻¹ MS (ES-) : 505 (M-H)⁻

### Beispiel 35: 3'-Chloro-4'-(4-phenoxy-5-formyl-2-phenyl-imidazol-1-ylmethyl)-biphenyl-2-sulfonylcyanamid

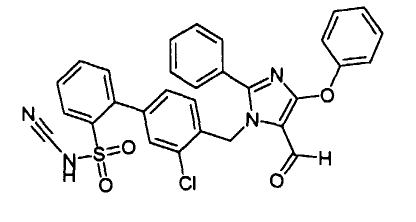

### a) 3'-Chloro-4'-(4-phenoxy-5-formyl-2-phenyl-imidazol-1-ylmethyl)-biphenyl-2-sulfonamid

500 mg 3'-Chloro-4'-(4-chloro-5-formyl-2-phenyl-imidazol-1-ylmethyl)-biphenyl-2-sulfonamid (Beispiel 32 d), 116 mg Phenol und 426 mg K₂CO₃ werden in 10 ml DMF 8 h bei 100 °C gerührt. Nach dem Abkühlen werden 100 ml einer gesättigten wäßrigen NaHCO₃-Lösung zugegeben und mit 300 ml EE extrahiert. Die organische Phase wird noch 3 mal mit je 50 ml Wasser gewaschen, über Na₂SO₄ getrocknet und das Solvens im Vakuum entfernt. Chromatographie an Kieselgel mit Toluol/EE 2:1 liefert 150 mg einer harzartigen Verbindung.
R_{f}(Toluol/EE 2:1) = 0.39 MS (ES+) : 544 (M+H)⁺

### b) 3'-Chloro-4'-(4-phenoxy-5-formyl-2-phenyl-imidazol-1-ylmethyl)-biphenyl-2-sulfonylcyanamid

145 mg 3'-Chloro-4'-(4-phenoxy-5-formyl-2-phenyl-imidazol-1-ylmethyl)-biphenyl-2-sulfonamid werden nach der allgemeinen Vorschrift zur Herstellung von Sulfonylcyanamiden aus Sulfonamiden umgesetzt (Reaktionszeit 2 h) und man erhält 99 mg eines Schaums.
R_{f}(EE/MeOH 10:1) = 0.35 IR (C≡N) : 2180.4 cm⁻¹ MS (ES-) : 567 (M-H)⁻

Die Tielverbindungen der Beispiele 36-38 werden analog Beispiel 11 synthetisiert:

### Beispiel 36: 4'-[4-Chloro-5-formyl-2-(4-chlorphenyl)-imidazol-1-ylmethyl]-biphenyl-2-sulfonylcyanamid

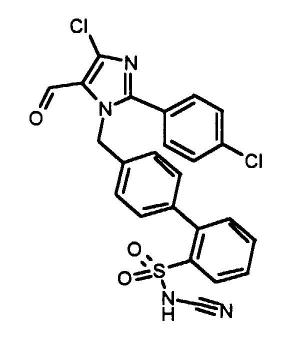

R_{f}(EE/MeOH 10:1) = 0.28 mp 160-187°C MS (ES) = 511 (M+1)⁺

### Beispiel 37: 4'-[4-Chloro-5-formyl-2-(4-methoxyphenyl)-imidazol-1-ylmethyl]-biphenyl-2-sulfonylcyanamid

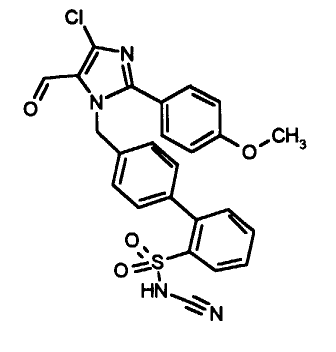

R_{f}(EE/MeOH 10:1) = 0.31 mp 110-126°C MS (ES) = 507 (M+1)⁺

### Beispiel 38: 4'-[4-Chloro-5-formyl-2-(2,6-difluorphenyl)-imidazol-1-ylmethyl]-biphenyl-2-sulfonylcyanamid

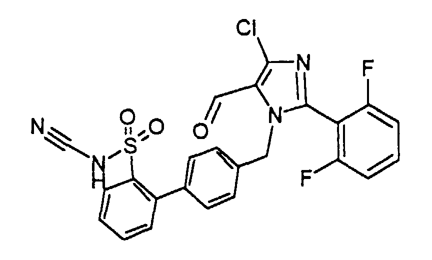

R_{f}(EE/MeOH 10:1) = 0.25 mp164-178°C MS (ES) = 513 (M+1)⁺

### Pharmakologische Daten:

### Inhibition des Na⁺- abhängigen Cl⁻/HCO₃⁻- Austauschers (NCBE) in humanen Endothelzellen

Humane Endothelzellen (ECV-304) wurden aus Kulturflaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCl, 20 NH₄Cl, 5 KCl, 1 CaCl₂, 1 MgSO₄, 20 N-(2-Hydroxyethyl)piperazin-N 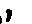 -2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37°C inkubiert.
Anschließend wurden die Zellen gewaschen und in einer Natrium- und Bicarbonatfreien Pufferlösung (mmol/l: 5 HEPES, 133,8 Cholinchlorid, 4,7 KCl, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min).
Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (PHᵢ) über den NCBE, eine Bicarbonat-und Natrium-haltige Pufferlösung (mmol/l: 5 HEPES, 93,8 NaCl, 40 NaHCO₃, 4,7 KCl, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCl, 4,7 KCl, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen wurden in verschiedenen Konzentrationen der Natrium-und Bicarbonat-haltigen Lösung zugesetzt.
Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem well der Mikrotiterplatte bestimmt. Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurde auf die beiden Kontrollen bezogen und daraus die Hemmwirkung der Substanzen ermittelt.

### Ergebnisse

| Restaktivität des NCBE bei einer Inhibitorkonzentration von 10 µM | |
|---|---|
| Beispiel | Restaktivität in % |
| 1 | 17.7 |
| 2 | 39.6 |
| 3 | 74.1 |
| 4 | 39.7 |
| 5 | 43.2 |
| 6 | 58.6 |
| 7 | 72.1 |
| 8 | 60.8 |
| 9 | 31.6 |
| 10 | 21.6 |
| 11 | 15.0 |
| 12 | 20.8 |
| 13 | 89.5 |
| 14 | 47.3 |
| 15 | 55.7 |
| 16 | 11.5 |
| 17 | 39.1 |
| 18 | 45.8 |
| 19 | 21.5 |
| 20 | 19.5 |
| 21 | 42.6 |
| 22 | 34.2 |
| 23 | 31.6 |
| 24 | 29.2 |
| 25 | 70.8 |
| 26 | 65.3 |
| 27 | 50.0 |
| 28 | 30.5 |
| 29 | 39.6 |
| 30 | 48.8 |
| 31 | 36.5 |
| 32 | 7.8 |
| 33 | 7.6 |
| 34 | 5.5 |
| 35 | 3.2 |
| 36 | 7.8 |
| 37 | 16.8 |
| 38 | 8.6 |

## Patentansprüche

1. Verbindungen der Formel I, 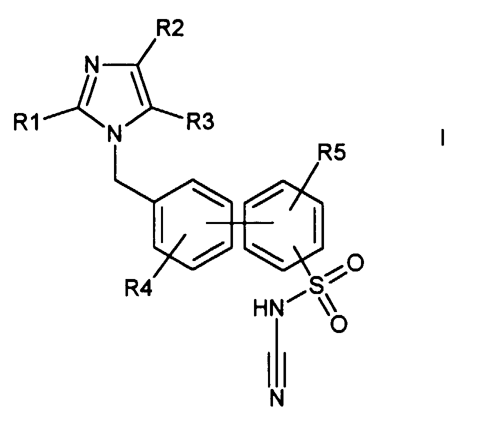 in welcher die Symbole folgende Bedeutung haben:
R1 Wasserstoff, Alkyl mit 1, 2, 3, 4, 5, 6, 7 oder 8 C-Atomen oder -CₐH₂ₐ-Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(8)R(9);
R(8) und R(9)
unabhängig voneinander H oder (C₁-C₄)-Alkyl;
a Null, 1 oder 2;
oder
R1 -C_{b}H_{2b}-(C₁-C₉)-Heteroaryl,
das unsubstituiert oder substituiert ist mit 1 - 3 Substituenten ausgewählt aus der Gruppe bestehend aus F, Cl, Br, I, CF₃, CH₃, Methoxy, Hydroxy und NR(10)R(11);
R(10) und R(11)
unabhängig voneinander H oder (C₁-C₄)-Alkyl;
b Null, 1 oder 2;
oder
R1 -C_{d}H_{2d}-(C₃-C₇)-Cycloalkyl;
d Null, 1 oder 2;
R2 und R3
unabhängig voneinander Wasserstoff, F, Cl, Br, I, CF₃, -C≡N, -NO₂, CH₂OR17, CO-R6 oder O-R7;
R17 Wasserstoff oder Alkyl mit 1, 2, 3, 4, 5, 6, 7 oder 8 C-Atomen
R6 Wasserstoff, Alkyl mit 1, 2, 3, 4, 5, 6, 7 oder 8 C-Atomen, OR30 oder Phenyl,
das unsubstituiert oder substituiert ist mit 1 - 3 Substituenten ausgewählt aus der Gruppe bestehend aus bestehend aus F, Cl, Br, I, CF₃, Methyl, Methoxy, Hydroxy und NR(31)R(32);
R(31) und R(32)
unabhängig voneinander H oder (C₁-C₄)-Alkyl;
R30 Wasserstoff oder Alkyl mit 1, 2, 3, 4, 5, 6, 7 oder 8 C-Atomen;
R7 Wasserstoff, Alkyl mit 1, 2, 3, 4, 5, 6, 7 oder 8 C-Atomen, Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(12)R(13);
R(12) und R(13)
unabhängig voneinander H oder (C₁-C₄)-Alkyl;
oder
R7 (C₁-C₉)-Heteroaryl,
das unsubstituiert oder substituiert ist mit 1 - 3 Substituenten ausgewählt aus der Gruppe bestehend aus F, Cl, Br, I, CF₃, CH₃, Methoxy, Hydroxy und NR(14)R(15);
R(14) und R(15)
unabhängig voneinander H oder (C₁-C₄)-Alkyl;
oder
R2 und R3
unabhängig voneinander Alkyl mit 1, 2, 3, 4, 5, 6, 7 oder 8 C-Atomen, Cycloalkyl mit 3, 4, 5, 6 oder 7 C-Atomen oder -C_{g}H₂g-Phenyl,
das unsubstituiert oder substituiert ist mit 1 - 3 Substituenten ausgewählt aus der Gruppe bestehend aus F, Cl, Br, I, CF₃, Methyl, Methoxy, Hydroxy und NR(18)R(19);
R(18) und R(19)
unabhängig voneinander H oder (C₁-C₄)-Alkyl;
g Null, 1 oder 2;
oder
R2 und R3
unabhängig voneinander -CₗH₂ₗ-(C₁-C₉)-Heteroaryl,
das unsubstituiert oder substituiert ist mit 1 - 3 Substituenten ausgewählt aus der Gruppe bestehend aus F, Cl, Br, I, CF₃, CH₃, Methoxy, Hydroxy und NR(20)R(21);
R(20) und R(21)
unabhängig voneinander H oder (C₁-C₄)-Alkyl;
l Null, 1 oder 2;
oder
R2 und R3
unabhängig voneinander SOₙ-R22;
n Null, 1 oder 2;
R22 Alkyl mit 1, 2, 3, 4, 5, 6, 7 oder 8 C-Atomen, Cycloalkyl mit 3, 4, 5, 6 oder 7 C-Atomen oder -CₛH₂ₛ-Phenyl,
das unsubstituiert oder substituiert ist mit 1 - 3 Substituenten ausgewählt aus der Gruppe bestehend Gruppe F, Cl, Br, I, CF₃, Methyl, Methoxy, Hydroxy und NR(34)R(35);
R(34) und R(35)
unabhängig voneinander H oder (C₁-C₄)-Alkyl;
s Null, 1 oder 2;
R4 und R5
unabhängig voneinander Wasserstoff, Alkyl mit 1, 2, 3, 4, 5, 6, 7 oder 8 C-Atomen, F, Cl, Br, I, CF₃, -C≡N, -NO₂, SOₚ-R16, CO-R23 oder O-R24;
p Null, 1 oder 2;
R16 Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, Br, I, CF₃, Methyl, Methoxy, Hydroxy und NR(26)R(27);
R(26) und R(27)
unabhängig voneinander H oder (C₁-C₄)-Alkyl;
R23 Wasserstoff, Alkyl mit 1, 2, 3, 4, 5, 6, 7 oder 8 C-Atomen oder OR25;
R25 Wasserstoff, Alkyl mit 1, 2, 3, 4, 5, 6, 7 oder 8 C-Atomen;
R24 Wasserstoff, Alkyl mit 1, 2, 3, 4, 5, 6, 7 oder 8 C-Atomen oder Phenyl, das unsubstituiert oder substituiert ist mit 1 - 3 Substituenten ausgewählt aus der Gruppe bestehend aus F, Cl, Br, I, CF₃, Methyl, Methoxy, Hydroxy und NR(28)R(29);
R(28) und R(29)
unabhängig voneinander H oder (C₁-C₄)-Alkyl;
sowie deren physiologisch verträgliche Salze.

2. Verbindung der Formel I nach Anspruch 1, in der bedeuten:
R1 Wasserstoff, Alkyl mit 1, 2, 3 oder 4 C-Atomen oder -CₐH₂ₐ-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(8)R(9);
R(8) und R(9)
unabhängig voneinander H oder Methyl;
a Null oder 1;
oder
R1 (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(10)R(11);
R(10) und R(11)
unabhängig voneinander H oder Methyl;
oder
R1 -C_{d}H_{2d}-(C₃-C₇)-Cycloalkyl;
d Null oder 1;
R2 und R3
unabhängig voneinander Wasserstoff, F, Cl, Br, CF₃, -C≡N, -NO₂, CH₂OR17, CO-R6 oder O-R7;
R17 Wasserstoff oder Alkyl mit 1, 2, 3 oder 4 C-Atomen;
R6 Wasserstoff, Alkyl mit 1, 2, 3 oder 4 C-Atomen, OR30 oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(31)R(32);
R(31) und R(32)
unabhängig voneinander H oder Methyl;
R30 Wasserstoff oder Alkyl mit 1, 2, 3 oder 4 C-Atomen
R7 Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, Br, CF₃, Methyl, Methoxy, Hydroxy und NR(12)R(13);
R(12) und R(13)
unabhängig voneinander H oder Methyl;
oder
R7 (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(14)R(15);
R(14) und R(15)
unabhängig voneinander H oder Methyl;
oder
R2 und R3
unabhängig voneinander Alkyl mit 1, 2, 3 oder 4 C-Atomen, Cycloalkyl mit 3, 4, 5, 6 oder 7 C-Atomen oder -C_{g}H_{2g}-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(18)R(19);
R(18) und R(19)
unabhängig voneinander H oder Methyl;
g Null oder 1;
oder
R2 und R3
unabhängig voneinander (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(20)R(21);
R(20) und R(21)
unabhängig voneinander H oder Methyl;
oder
R2 und R3
unabhängig voneinander SOₙ-R22,
n Null, 1 oder 2;
R22 Alkyl mit 1, 2, 3 oder 4 C-Atomen, Cycloalkyl mit 3, 4, 5, 6 oder 7 C-Atomen oder -CₛH₂ₛ-Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(34)R(35);
R(34) und R(35)
ausgewählt aus der Gruppe bestehend aus H oder Methyl;
s Null oder 1;
R4 und R5
unabhängig voneinander Wasserstoff, Alkyl mit 1, 2, 3 oder 4 C-Atomen, F, Cl, Br, CF₃, -C≡N, -NO₂, SOₚ-R16, CO-R23 oder O-R24;
p Null, 1 oder 2;
R¹⁶ Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(26)R(27);
R(26) und R(27)
unabhängig voneinander H oder Methyl;
R23 Wasserstoff, Alkyl mit 1, 2, 3 oder 4 C-Atomen oder OR25;
R25 Wasserstoff oder Alkyl mit 1, 2, 3 oder 4 C-Atomen;
R24 Wasserstoff, Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(28)R(29);
R(28) und R(29)
unabhängig voneinander H oder Methyl;
sowie deren physiologisch verträglichen Salze.

3. Verbindung der Formel I nach Anspruch 1, in der bedeuten:
R1 Wasserstoff, Alkyl mit 1, 2, 3 oder 4 C-Atomen oder Phenyl, das unsubstituiert oder substituiert ist mit einem Substituenten ausgewählt aus der Gruppe bestehend aus F, Cl, Br, CF₃, Methyl, Methoxy, Hydroxy und NR(8)R(9);
R(8) und R(9)
unabhängig voneinander H oder Methyl;
oder
R1 (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(10)R(11);
R(10) und R(11)
unabhängig voneinander H oder Methyl;
oder
R1 (C₃-C₇)-Cycloalkyl;
R2 und R3
unabhängig voneinander Wasserstoff, F, Cl, Br, CF₃, -C≡N, -NO₂, CO-R6 oder O-R7;
R6 Wasserstoff, Alkyl mit 1, 2, 3 oder 4 C-Atomen, OR30 oder Phenyl, das unsubstituiert oder substituiert ist mit einem Substituenten ausgewählt aus der Gruppe bestehend aus F, Cl, Br, CF₃, Methyl, Methoxy, Hydroxy und NR(31)R(32);
R(31) und R(32)
unabhängig voneinander H oder Methyl;
R30 Wasserstoff oder Alkyl mit 1, 2 oder 3 C-Atomen
R7 Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(12)R(13);
R(12) und R(13)
unabhängig voneinander H oder Methyl;
oder
R7 (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(14)R(15); R(14) und R(15)
unabhängig voneinander H oder Methyl;
oder
R2 und R3
unabhängig voneinander Alkyl mit 1, 2, 3 oder 4 C-Atomen, Cycloalkyl mit 3, 4, 5, 6 oder 7 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(18)R(19);
R(18) und R(19)
unabhängig voneinander H oder Methyl;
oder
R2 und R3
unabhängig voneinander (C₁-C₉)-Heteroaryl,
das unsubstituiert oder substituiert ist mit einem Substituenten ausgewählt aus der Gruppe bestehend aus F, Cl, Br, CF₃, CH₃, Methoxy, Hydroxy und NR(20)R(21);
R(20) und R(21)
unabhängig voneinander H oder Methyl;
oder
R2 und R3
unabhängig voneinander SOₙ-R22;
n Null oder 2;
R22 Alkyl mit 1, 2, 3 oder 4 C-Atomen, Cycloalkyl mit 3, 4, 5, 6 oder 7 C-Atomen oder Phenyl,
das unsubstituiert oder substituiert ist mit 1 - 2 Substituenten ausgewählt aus der Gruppe bestehend aus F, Cl, Br, CF₃, Methyl, Methoxy, Hydroxy und NR(34)R(35);
R(34) und R(35)
unabhängig voneinander H oder Methyl;
R⁴ und R⁵ unabhängig voneinander
Wasserstoff, Alkyl mit 1, 2, 3 oder 4 C-Atomen, F, Cl, CF₃, -C≡N, -NO₂, SOₚ-R16, CO-R23 oder O-R24;
p Null oder 2;
R23 Wasserstoff, Alkyl mit 1, 2, 3 oder 4 C-Atomen oder OR25;
R25 Wasserstoff oder Alkyl mit 1, 2 oder 3 C-Atomen;
R24 Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(28)R(29);
R(28) und R(29)
unabhängig voneinander H oder Methyl;
R16 Alkyl mit 1, 2, 3 oder 4 C-Atomen oder Phenyl,
das unsubstituiert oder substituiert ist mit einem Substituenten ausgewählt aus der Gruppe bestehend aus F, Cl, Br, CF₃, Methyl, Methoxy, Hydroxy und NR(26)R(27);
R(26) und R(27)
unabhängig voneinander H oder Methyl;
sowie deren physiologisch verträglichen Salze.

4. Verbindung der Formel I nach Anspruch 1, in der bedeuten:
R1 Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
oder
R1 (C₁-C₉)-Heteroaryl,
das unsubstituiert oder substituiert ist mit einem Substituenten ausgewählt aus der Gruppe bestehend aus F, Cl, CF₃, CH₃ und Methoxy;
oder
R1 (C₃-C₇)-Cycloalkyl;
R2 und R3
unabhängig voneinander Wasserstoff, F, Cl, CF₃, -C≡N, CO-R6 oder O-R7;
R6 Wasserstoff, Alkyl mit 1, 2, 3 oder 4 C-Atomen, OR30 oder Phenyl,
das unsubstituiert oder substituiert ist mit einem Substituenten ausgewählt aus der Gruppe bestehend aus F, Cl, CF₃, Methyl und Methoxy;
R30 Wasserstoff, Methyl oder Ethyl;
R7 Wasserstoff, Alkyl mit 1, 2, 3 oder 4 C-Atomen oder Phenyl, das unsubstituiert oder substituiert ist mit einem Substituenten ausgewählt aus der Gruppe bestehend aus F, Cl, CF₃, Methyl und Methoxy;
oder
R7 (C₁-C₉)-Heteroaryl,
das unsubstituiert oder substituiert ist mit einem Substituenten ausgewählt aus der Gruppe bestehend aus F, Cl, Br, CF₃, CH₃ und Methoxy;
oder
R2 und R3
unabhängig voneinander Alkyl mit 1, 2, 3 oder 4 C-Atomen, Cycloalkyl mit 3, 4, 5, 6 oder 7 C-Atomen oder Phenyl,
das unsubstituiert oder substituiert ist mit einem Substituenten ausgewählt aus der Gruppe bestehend aus F, Cl, CF₃, Methyl und Methoxy;
oder
R2 und R3
unabhängig voneinander (C₁-C₉)-Heteroaryl,
das unsubstituiert oder substituiert ist mit einem Substituenten ausgewählt aus der Gruppe bestehend aus F, Cl, CF₃, CH₃ und Methoxy;
oder
R2 und R3
unabhängig voneinander SOₙ-R22;
n Null oder 2;
R22 Alkyl mit 1, 2, 3 oder 4 C-Atomen oder Phenyl,
das unsubstituiert oder substituiert ist mit 1 - 2 Substituenten ausgewählt aus der Gruppe bestehend aus F, Cl, CF₃, Methyl und Methoxy;
R4 und R5
unabhängig voneinander Wasserstoff, Methyl, F, Cl, CF₃, -C≡N, SO₂-R16, CO-R23 oder O-R24;
R¹⁶ Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
R23 Wasserstoff, Methyl oder OR25;
R25 Wasserstoff, Methyl oder Ethyl;
R24 Wasserstoff, Alkyl mit 1, 2, 3 oder 4 C-Atomen oder Phenyl,
das unsubstituiert oder substituiert ist mit einem Substituenten ausgewählt aus der Gruppe bestehend aus F, Cl, CF₃, Methyl und Methoxy;
sowie deren physiologisch verträglichen Salze.

5. Verbindung der Formel I nach einem oder mehreren der Ansprüche 1-4, dadurch gekennzeichnet, daß der Biphenylsubstituent wie in Formel Ia, Ib, Ic, Id, Ie, If, Ig oder Ih verknüpft ist 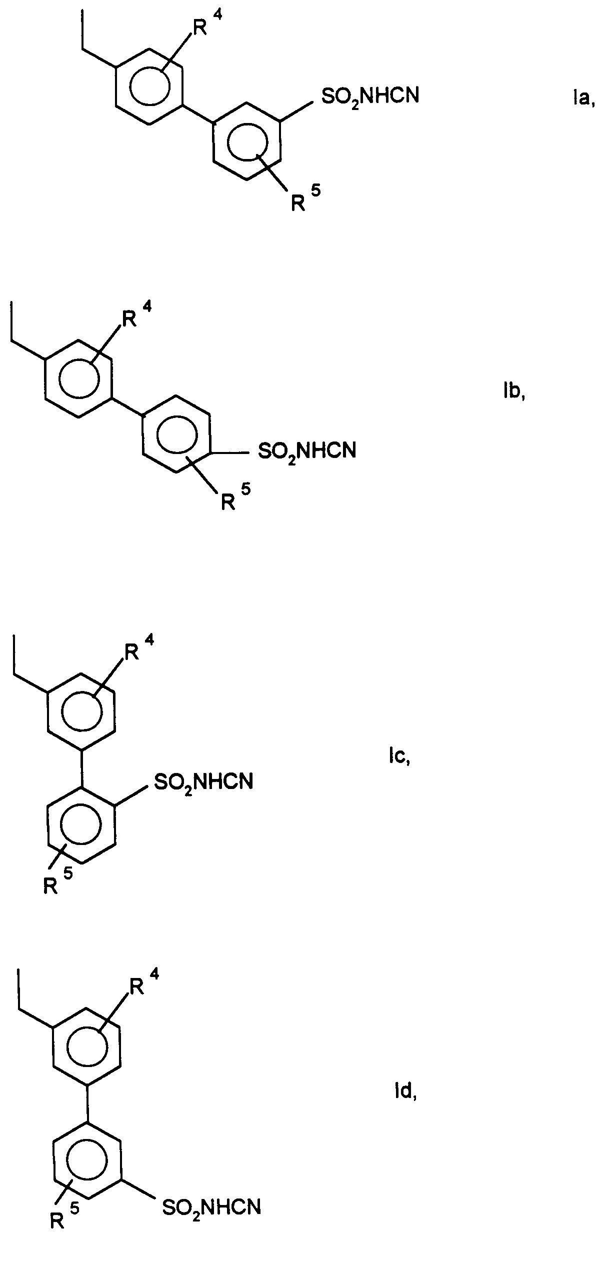 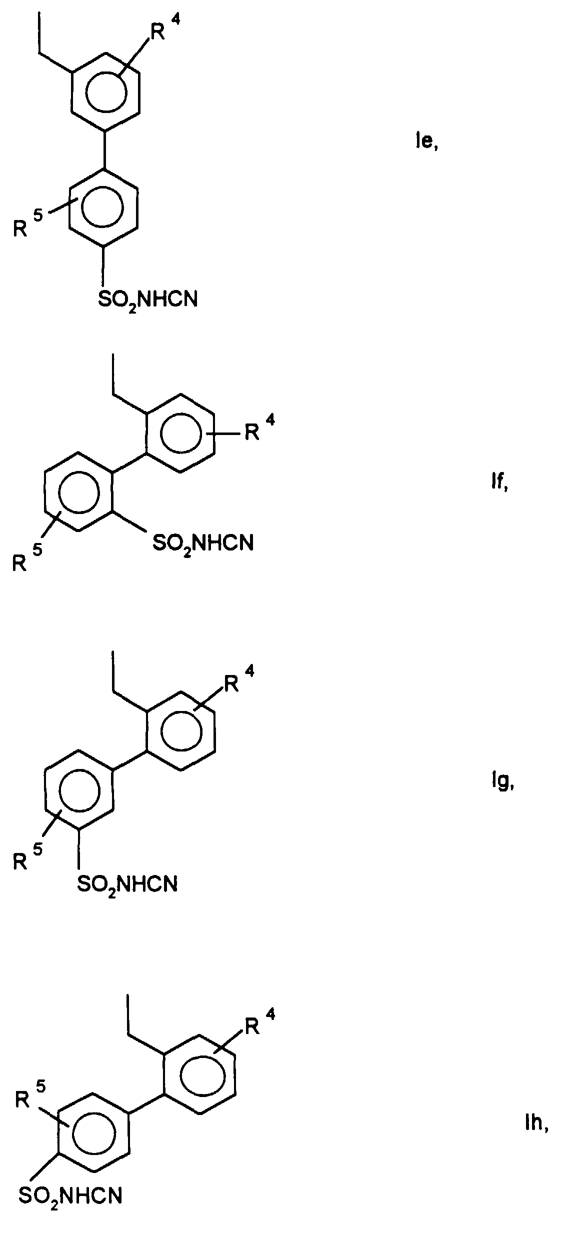 wobei R⁴ und R⁵ wie in Anspruch 1, 2, 3 oder 4 definiert ist, sowie deren physiologisch verträglichen Salze.

6. Verwendung einer Verbindung der Formel 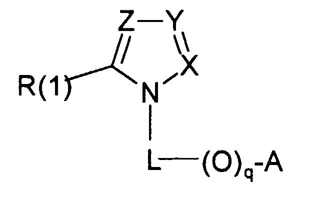 in welcher bedeuten:
a) X, Y und Z
gleich oder verschieden, N oder CR(102)
b) R(1)
1. (C₁-C₁₀)-Alkyl, 2. (C₃-C₁₀)-Alkenyl, 3. (C₃-C₁₀)-Alkinyl, 4. -OR(103), 5. (C₃-C₈)-Cycloalkyl, 6. (C₄-C₁₀)-Cycloalkylalkyl, 7. (C₅-C₁₀)-Cycloalkylalkenyl, 8. (C₅-C₁₀)-Cycloalkylalkinyl, 9. -(CH₂)ₘ-B-(CH₂)ₙ-R(104), 10. -Benzyl, 11. ein wie unter b) 1., 2., 3. oder 9. definierter Rest, der mit CO₂R(103) monosubstituiert ist, 12. ein wie unter b) 1., 2., 3. oder 9. definierter Rest, worin 1 bis alle H-Atome durch Fluor substituiert sind, oder 13. den unter b) 10. definierten Rest, der am Phenyl mit 1 oder 2 gleichen oder verschiedenen Resten aus der Reihe Halogen, (C₁-C₄)-Alkoxy und Nitro substituiert ist,
c) R(102)
1. Wasserstoff, 2. Halogen, 3. Nitro, 4. CᵥF₂ᵥ₊₁, 5. Pentafluorphenyl, 6. Cyano, 7. -O-R(106), 8. Phenyl, 9. Phenyl-(C₁-C₃)-alkyl, 10. (C₁-C₁₀)-Alkyl, 11. (C₃-C₁₀)-Alkenyl, 12. Phenyl-(C₂-C₆)-Alkenyl, 13. 1-Imidazolyl-(CH₂)ₘ-, 14. 1,2,3-Triazolyl-(CH₂)ₙ-, 15. Tetrazolyl-(CH₂)ₘ-, 16. -(CH2)ₒ₋₁-CHR(107)-OR(105), 17. -(CH₂)ₒ-O-CO-R(103), 18. - (CH₂)ₒ-S-R(106), 19. -S(O)ᵣ-R(119), 20. CH=CH-(CH₂)ₘ-CHR(103)-OR(106), 21. -CH=CH(CH₂)ₘ-CO-R(108), 22. -CO-R(108), 23. - CH=CH-(CH₂)ₘ-O-CO-R(107), 24. -(CH₂)ₘ-CH(CH₃)-CO-R(108), 25. - (CH₂)ₒ-CO-R(108), 26. -(CH₂)ₒ-O-[C=W]-NH-R(109), 27. -(CH₂)ₒ-NR(107)-[C=W]-OR(109), 28. -(CH₂)ₒ-NR(107)-CO-NHR(109), 29. - (CH₂)ₒ-NR(107)-SO₂R(109), 30. -(CH₂)ₒ-NR(107)-[C=W]-R(109), 31. - (CH₂)ₙF, 32. -(CH₂)ₙ-O-NO₂, 33. -CH₂-N₃, 34. -(CH₂)ₙ-NO₂, 35. -CH=N-NR(105)R(107), 36. Phthalimido-(CH₂)ₙ-,
37. 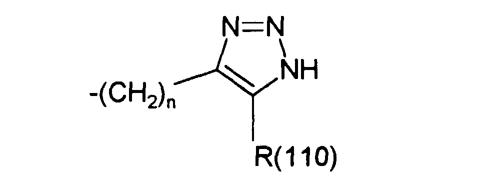
38. 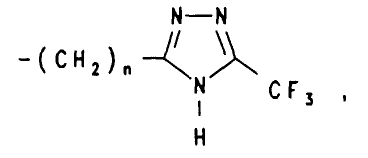
39. 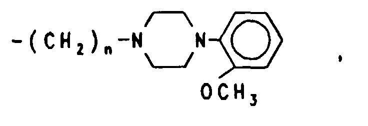
40. 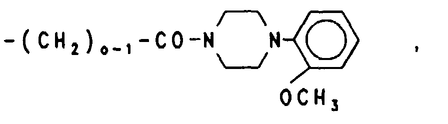
41. 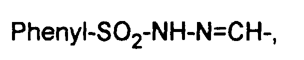
42. 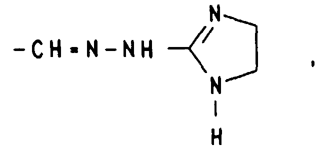
43. -(CH₂)ₙ-SO₂-NR(107)-CS-NR(106)R(109), 44. -(CH₂)ₙSO₂-NR(107)-CO-NR(106)R(109), 45. -(CH₂)ₒ-SO₂R(109), 46. einen wie unter c) 8. oder 9. definiertem Rest, der am Phenyl mit 1 oder 2 gleichen oder verschiedenen Resten aus der Reihe Halogen, Hydroxy, Methoxy, Trifluormethyl, CO₂R(103) und Phenyl substituiert ist, 47. einem wie unter c) 10., 11. oder 19. definierten Rest, worin ein bis alle H-Atome durch Fluor substituiert sind, 48. den unter c) 14. definierten Rest, der mit 1 oder 2 gleichen oder verschiedenen Resten aus der Reihe Methoxycarbonyl und (C₁-C₄)-Alkyl substituiert ist, 49. -(CH₂)ₙ-SO₂-NR(107)-CO-R(106), 50. -(CH₂)ₙ-SO₂-NR(107)CS-R(106),
d) R(103)
1. Wasserstoff, 2. (C₁-C₈)-Alkyl, 3. (C₃-C₈)-Cycloalkyl, 4. Phenyl, 5. Benzyl, 6. den unter d) 2. definiertem Rest, worin 1 bis alle H-Atome durch Fluor substituiert sind;
e) R(104)
1. Wasserstoff, 2. (C₁-C₆)-Alkyl, 3. (C₃-C₈)-Cycloalkyl, 4. (C2-C₄)-Alkenyl oder 5. (C₂-C₄)-Alkinyl;
f) R(105)
1. Wasserstoff, 2. (C₁-C₆)-Alkyl, 3. (C₃-C₈)-Cycloalkyl, 4. Phenyl oder 5. Benzyl;
g) R(106) und R(109)
gleich oder verschieden 1. Wasserstoff, 2. (C₁-C₆)-Alkyl, das unsubstituiert ist oder substituiert mit 1 - 3 Substituenten ausgewählt aus der Gruppe bestehend aus (C₁-C₆)-Alkoxy, das seinerseits mit 1 - 3 Resten aus der Reihe Hydroxy, (C₁-C₆)-Alkoxy, Amino, Mono-(C₁-C₆)-Alkylamino, Di-(C₁-C₆)-Alkylamino substituiert sein kann, (C₂-C₁₀)-Alkenyl, Hydroxy, Amino, Mono-(C₁-C₆)-Alkylamino, Di-(C₁-C₆)-Alkylamino, (C₁-C₆)-Alkoxycarbonylamino, (C₆-C₁₂)-Aryl-(C₁-C₄)Alkoxycarbonylamino; (C₆-C₁₀)-Aryl, (C₆-C₁₀)Aryl-(C₁-C₃)-Alkyl, (C₁-C₉)-Heteroaryl, Carboxy und (C₁-C₄)-Alkoxycarbonyl; 3. (C₃-C₈)-Cycloalkyl,
wobei der Cycloalkylteil unsubstituiert ist oder substituiert mit 1 - 3 Substituenten ausgewählt aus der Gruppe bestehend aus (C₁-C₄)-Alkyl und (C₂-C₄)-Alkenyl;
4. (C₃-C₈)-Cycloalkyl-(C₁-C₃)-alkyl, 5. (C₆-C₁₂)-Aryl, vorzugweise Phenyl, 6. (C₆-C₁₀)-Aryl-(C₁-C₄)-Alkyl, 7. (C₁-C₉)-Heteroaryl,
welches teilweise oder vollständig hydriert sein kann,
8. einen wie unter g) 5., 6., 7., 9., 15., 16., 17., 19., 20. oder 21. definiertem Rest, substituiert mit 1 oder 2 gleichen oder verschiedenen Resten aus der Reihe Halogen, Hydroxy, (C₁-C₄)-Alkyl, Methoxy, Nitro, Cyano, CO₂R(103), Trifluormethyl, NR(111)R(112) und 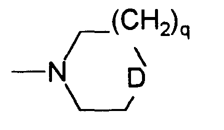
9. (C₁-C₉)-Heteroaryl-(C₁-C₃)-alkyl,
wobei der Heteroarylteil teilweise oder vollständig hydriert sein kann,
10. (C₁-C₆)-Alkyl, worin 1 bis alle H-Atome durch Fluor substituiert sind,
11. (C₂-C₁₀)-Alkenyl, (C₂-C₁₀)-Alkenoyl oder (C₂-C₁₀)-Alkadienyl, 12. (C₃-C₈)-Cycloalkenyl, 13. (C₃-C₈)-Cycloalkenyl-(C₁-C₃)-Alkyl, 14. Bi- oder
tricyclisches (C₄-C₁₀)-Cycloalkenyl-(C₁-C₄)-alkyl, das noch mit 1 - 3 (C₁-C₄)-Alkylresten substituiert sein kann;
15. (C₆-C₁₀)-Aryl-(C₁-C₄)-Alkyl, 16. (C₆-C₁₀)-Aryl-(C₃-C₆)-Alkenyl, 17. (C₁-C₉)-Hetaryl-(C₃-C₆)-Alkenyl, 18. (C₃-C₆)-Alkinyl, 19. (C₆-C₁₀)-Aryl-(C₃-C₆)-Alkinyl, 20. (C₁-C₉)-Hetaryl-(C₃-C₆)-Alkinyl, 21. R(106) und R(109) gemeinsam mit dem sie tragenden N-Atom ein Hetaryl, das auch teilweise oder vollständig hydriert sein kann;
h) R(107)
1. Wasserstoff, 2. (C₁-C₆)-Alkyl, 3. (C₃-C₈)-Cycloalkyl, 4. (C₆-C₁₂)-Aryl-(C₁-C₆)-alkyl, vorzugsweise Benzyl, 5. Phenyl oder 6. (C₁-C₉)-Heteroaryl;
i) R(108)
1. Wasserstoff, 2. (C₁-C₆)-Alkyl, 3. (C₃-C₈)-Cycloalkyl, 4. Phenyl-(CH₂)_{q}-, 5. OR(106), 6. NR(111)R(112) oder 7. 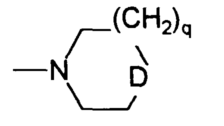
j) R(110)
Cyano, Nitro oder CO₂R(107);
k) R(111) und R(112)
gleich oder verschieden, 1. Wasserstoff, 2. (C₁-C₄)-Alkyl, 3. Phenyl, 4. Benzyl, oder 5. α-Methylbenzyl;
l) D NR(113), O oder CH₂;
m) R(113)
Wasserstoff, (C₁-C₄)-Alkyl oder Phenyl;
n) A Biphenylyl,
das unsubstituiert ist oder substituiert mit 1 - 4, vorzugsweise 1 bis 2 gleichen oder verschiedenen Substituenten R(114) oder R(115);
o) R(114)
1. Halogen, 2. Nitroso, 3. Nitro, 4. Amino, 5. Cyano, 6. Hydroxy, 7. (C₁-C₆)-Alkyl, 8. (C₁-C₄)-Alkanoyl, 9. (C₁-C₄)-Alkanoyloxy, 10. CO₂R(103), 11. Methansulfonylamino, 12. Trifluormethansulfonylamino, 13. -CO-NH-OR(109), 14. -SO₂-NR(106)R(107), 15. -CH₂-OR(107), 16. (C₁-C₉)-Heteroaryl-(CH₂)_{q}-, vorzugsweise 1-Tetrazolyl, 17. (C₇-C₁₃)-Aroyl,
18. 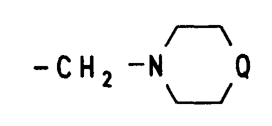
19. 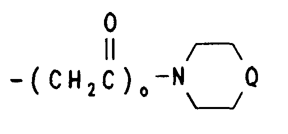 oder
20. (C₆-C₁₂)-Aryl;
p) R(115)
1. Wasserstoff, 2. (C₁-C₆)-Alkyl, 3. (C₃-C₈)-Cycloalkyl, 4. (C₆-C₁₂)-Aryl, 5. (C₇-C₁₃)-Aroyl, 6. (C₁-C₄)-Alkoxy, 7. (C₁-C₄)-Alkanoyloxy, 8. (C₁-C₉)-Heteroaryl, 9. CO₂R(103), 10. Halogen, 11. Cyano, 12. Nitro, 13. NR(106)R(107), 14. Hydroxy, 15. -CO-NH-CHR(105)-CO₂R(103), 16. Sulfo, 17. -SO₃R(103), 18. -SO₂-NR(107)-CO-NR(106)R(109) oder -SO₂-NR(107)-CS-NR(106)R(109), 19. -NR(107)-CO-NR(106)-SO₂-CH₂-R(105), 20. -C(CF₃)₂OH, 21. Phosphonooxy, 22. -PO₃H₂, 23. -NH-PO(OH)₂, 24. -S(O)ᵣR(106), 25. -CO-R(108), 26. -CO-NR(106)R(109), 27. -CR(120)(OH)-PO(OH)₂, 28. den unter o) 20. definiertem Rest,
29. 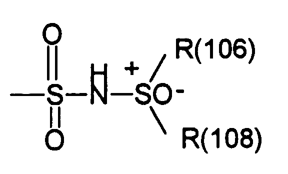
30. 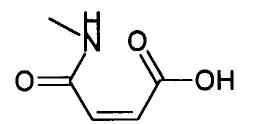
31. 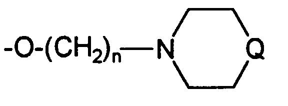
32. 5-Tetrazolyl-NH-CO-, 33. -CO-NH-NH-SO₂-CF₃,
34. 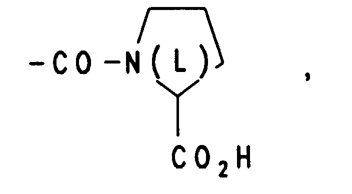
35. 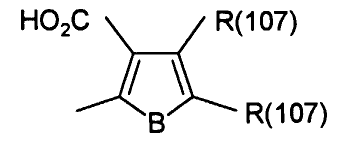
36. 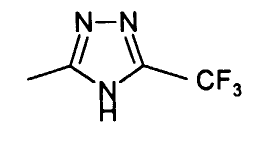
37. 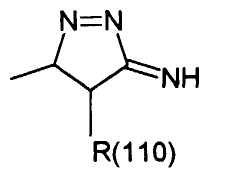
38. 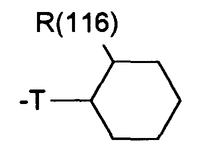
39. 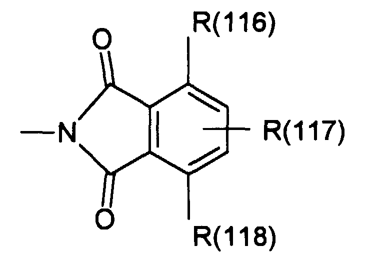
40. -CO-NH-SO₂-R(119), 41. -SO₂-NH-CO-R(106) oder 42. den unter p) 4. definiertem Rest, substituiert mit 1 oder 2 gleichen oder verschiedenen Resten aus der Reihe Halogen, Cyano, Nitro, NR(106)R(107) und Hydroxy; 43. R(115) zusammen mit R(114) bedeutet -CO-NH-SO₂-, 44. -SO₂-NH-CO-O-R(106), 45. -SO₂-NH-SO₂-NR(106)R(109), 46. -SO₂-NH-SO₂-R(106);
q) B O, NR(107) oder S;
r) W O oder S;
s) L (C₁-C₃)-Alkandiyl;
t) R(116)
CO₂R(103) oder CH₂CO₂R(103);
u) R(117)
Wasserstoff, Halogen, (C₁-C₄)-Alkyl oder (C₁-C₄)-Alkoxy;
v) R(118)
Wasserstoff, (C₁-C₄)-Alkyl oder Phenyl;
w) R(119)
1. (C₁-C₆)-Alkyl, 2. (C₃-C₈)-Cycloalkyl, 3. Phenyl, 4. Benzyl oder 5. den unter w) 1. definiertem Rest, worin ein bis all H-Atome durch Fluor substituiert sind,
x) T
1. eine Einfachbindung, 2. -CO-, 3. -CH₂-, 4. -O-, 5. -S-, 6. -NR(121)-, 7. -CO-NR(121), 8. -NR(121)-CO-, 9. -O-CH₂-, 10. -CH₂-O-, 11. -S-CH₂-, 12. -CH₂-S, 13. -NH-CR(120)R(122), 14. -NR(121)-SO₂, 15. SO₂-NR(121)-, 16. -CR(120)R(122)-NH, 17. -CH=CH-, 18. -CF=CF-, 19. -CH=CF-, 20. -CF=CH-, 21. -CH₂-CH₂-, 22. -CF₂-CF₂-, 23. -CH[OR(103)]-, 24. -CH(OCOR(105))-, 25. -C[N=R(123)]- oder 26. -[R(124)O]-C-[OR(125)]-
y) R(120) und R(122)
gleich oder verschieden, Wasserstoff, (C₁-C₅)-Alkyl, Phenyl, Allyl oder Benzyl;
z) R(121)
Wasserstoff, (C₁-C₆)-Alkyl, Benzyl oder Allyl; a') R(123)
1. NR(120)R(121), 2. Ureido, 3. Thioureido, 4. Toluol-4-sulfonyl oder 5. Benzolsulfonylamino;
b') R(124) und R(125)
gleich oder verschieden, (C₁-C₄)-Alkyl oder gemeinsam -(CH₂)_{q}-;
c') Q CH₂, NH, O oder S;
d') m 1, 2, 3, 4 oder 5;
e') n 1, 2, 3, 4 oder 5;
f') o 1, 2, 3, 4, 5, 6, 7, 8, 9 oder 10;
g') q Null oder 1;
h') r Null, 1 oder 2;
i') v 1, 2, 3, 4, 5 oder bis 6;
sowie deren physiologisch verträgliche Salze zur Herstellung eines Medikaments zur Behandlung oder Prophylaxe von durch ischämische Zustände hervorgerufenen Krankheiten oder zur Herstellung eines Medikaments zur Behandlung von gestörtem Atemantrieb.

7. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von durch ischämische Zustände hervorgerufenen Krankheiten oder zur Herstellung eines Medikaments zur Behandlung von gestörtem Atemantrieb.

8. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von durch ischämische Zustände hervorgerufenen Krankheiten.

9. Verwendung einer Verbindung der Formel I nach einem oder mehreren der Ansprüche 1-5 sowie deren physiologisch verträglichen Salzen zur Herstellung eines Medikaments zur Behandlung oder Prophylaxe des Herzinfarkts.

10. Verwendung einer Verbindung der Formel I nach einem oder mehreren der Ansprüche 1-5 sowie deren physiologisch verträglichen Salzen zur Herstellung eines Medikaments zur Behandlung oder Prophylaxe der Angina Pectoris.

11. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von ischämischen Zuständen des Herzens.

12. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von ischämischen Zuständen des peripheren und zentralen Nervensystems und des Schlaganfalls.

13. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von ischämischen Zuständen peripherer Organe und Gliedmaßen.

14. Verwendung einer Verbindung der Formel I nach einem oder mehreren der Ansprüche 1-5 sowie deren physiologisch verträglichen Salzen zur Herstellung eines Medikaments zur Behandlung von Schockzuständen.

15. Verwendung einer Verbindung der Formel I nach einem oder mehreren der Ansprüche 1-5 sowie deren physiologisch verträglichen Salzen zur Herstellung eines Medikaments zum Einsatz bei chirurgischen Operationen und Organtransplantationen.

16. Verwendung einer Verbindung der Formel I nach einem oder mehreren der Ansprüche 1-5 sowie deren physiologisch verträglichen Salzen zur Herstellung eines Medikaments zur Konservierung und Lagerung von Transplantaten für chirurgische Maßnahmen.

17. Verwendung einer Verbindung der Formel I nach einem oder mehreren der Ansprüche 1-5 sowie deren physiologisch verträglichen Salzen zur Herstellung eines Medikaments zur Behandlung von Krankheiten, bei denen die Zellproliferation eine primäre oder sekundäre Ursache darstellt.

18. Verwendung einer Verbindung der Formel I nach einem oder mehreren der Ansprüche 1-5 sowie deren physiologisch verträglichen Salzen zur Herstellung eines Medikaments zur Behandlung oder Prophylaxe von gestörtem Atemantrieb.

19. Arzneimittel, gekennzeichnet durch einen wirksamen Gehalt einer Verbindung der Formel I sowie deren physiologisch verträglichen Salze gemäß einem oder mehreren der Ansprüche 1 bis 5.

20. Arzneimittel gemäß Anspruch 19, dadurch gekennzeichnet, daß es zusätzlich eine wirksame Menge eines NHE-Inhibitors und/oder eine wirksame Substanz aus einer anderen Herz-Kreislauf-Wirkstoffklasse, oder deren physiologisch verträglichen Salze enthält.
